# FASCICULE DE BREVET EUROPEEN

(11) **EP 0 776 885 B1**
(45) Date de publication et mention de la délivrance du brevet: **27.10.1999**
(21) Numéro de dépôt: 96402424.4
(22) Date de dépôt: 13.11.1996
(51) Int. Cl.: C07C 323/56, C07C 317/44, C07C 327/48, C07C 323/62, C07C 229/60, C07C 65/40, C07C 235/56, C07C 69/92, C07C 65/28, C07C 327/32, C07D 317/22, C07D 333/38, A61K 7/48, A61K 7/06, A61K 31/19

(54) **Composés biaromatiques portant un groupement adamantyl en ortho, compositions pharmaceutiques et cosmétiques les contenant et utilisations**
Mit ortho-Adamantylgruppe substituierte diaromatische Verbindungen, diese enthaltende pharmazeutische und kosmetische Zusammensetzungen und Verwendungen
Diaromatic compounds with an adamantyl group in the ortho position, pharmaceutical and cosmetic compositions containing them and uses

(30) Priorité: 01.12.1995 FR 9514260
(43) Date de publication de la demande: 04.06.1997
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Bernardon, Jean-Michel, 06650 Le Rouret (FR)
(74) Mandataire: Tezier Herman, Béatrice

(56) Documents cités:
- EP-A- 0 232 199
- EP-A- 0 325 540
- EP-A- 0 409 728
- EP-A- 0 409 729
- EP-A- 0 658 553
- EP-A- 0 679 631
- FR-A- 2 601 002
- JOURNAL OF MEDICINAL CHEMISTRY, vol. 38, no. 26, 22 Décembre 1995, WASHINGTON, DC, US, pages 4993-5006, XP002026831 B. CHARPENTIER, ET AL.: "Synthesis, structure-affinity relationships, and biological activities of ligands binding to retinoic acid receptor subtypes"

## Description

L'invention concerne, à titre de produits industriels nouveaux et utiles, des composés biaromatiques. Elle concerne également l'utilisation de ces nouveaux composés dans des compositions pharmaceutiques destinées à un usage en médecine humaine ou vétérinaire, ou bien encore dans des compositions cosmétiques.

Dans le document EP-A-232199 (D1), il a été décrit des composés benzamido aromatiques qui peuvent correspondre aux composés de formule (I) présentés ci-après dans lesquels X représente (c), Ar représente (a), R3 représente un radical hydroxy ou un radical O-alkyle en C1-C10. Ces composés sont exclus de la présente invention par la condition (1) du disclaimer qui précise que lorsque X représente la liaison de formule (c), alors R₃ est différent du radical de formule (iii) dans laquelle R₈ est un atome d'hydrogène.

Dans le document EP-A-325540 (D2), il a été décrit des composés qui peuvent correspondre aux composés de formule (I) présentés ci-après dans lesquels X représente (a) ou (b), Ar représente (a), R3 représente un radical hydroxy ou un radical O-alkyle inférieur. Ces composés sont exclus de la présente invention par la condition (1) du disclaimer qui précise que lorsque X représente la liaison de formule (a) ou (b), alors R₃ est différent du radical de formule (iii) dans laquelle R₈ est un atome d'hydrogène.

Dans le document EP-A-409728 (D3), il a été décrit des composés qui peuvent correspondre aux composés de formule (I) présentés ci-après dans lesquels X représente (a), Ar représente (a), R3 représente un atome d'hydrogène, un radical alkyle en C1-C6, un radical alkényle, un radical alkényle-oxy, un radical O-R12, S-R13, SOR14, SO2R14, avec
R12 représente un atome d'hydrogène, un radical alkyle en C1-C6, un radical mono ou polyhydroxyalkyle, ou un radical -(CH2)n-COR15;
R13 représente un atome d'hydrogène, ou un radical alkyle en C1-C6;
R14 représente un radical hydroxy ou un radical alkyle en C1-C6;
Ces composés sont exclus de la présente invention par les conditions (1) et (2) du disclaimer qui précisent que lorsque X représente la liaison de formule (a), alors R₃ est différent du radical de formule (iii), (iv) ou (v) dans laquelle R₈ est un atome d'hydrogène, et lorsque X représente la liaison de formule (a), alors R₃ est différent du radical (iii).

Dans le document EP 409729 (D4), il a été décrit des composés qui peuvent correspondre aux composés de formule (I) présentés ci-après dans lesquels X représente (b), Ar représente (a), R3 représente un atome d'hydrogène, un radical alkyle en C1-C6, un radical alkényle, un radical alkényle-oxy, un radical O-R12, S-R13,
R12 représente un atome d'hydrogène, ou un radical alkyle en C1-C6;
R13 représente un atome d'hydrogène, un radical alkyle en C1-C6, ou un radical aralkyle;
Ces composés sont exclus de la présente invention par les conditions (1) et (3) du disclaimer qui précisent que lorsque X représente (b), alors R₃ est différent du radical de formule (iii), (iv) ou (v) dans laquelle R₈ est un atome d'hydrogène, et lorsque X représente la liaison de formule (b) et R₃ représente le radical de formule (iii) dans laquelle Y représente un atome de soufre, alors R₈ est différent du radical aryle.

Dans le document EP-A-552282, il a été décrit des composés qui peuvent correspondre aux composés de formule (I) présentés ci-après dans lesquels X représente (g), R3 représente un atome d'hydrogène, un radical hydroxy, un radical alkoxy en C1-C6, un radical alkyle α-substitué en C3-C12, un radical cycloalkyle ayant de 3 à 12 atomes de carbone, un radical alkényle ayant de 2 à 6 atomes de carbone, un radical mono ou polyhydryalkyle, un radical -SH, S-alkyle inférieur, SO-alkyle inférieur, SO2-alkyle inférieur.
Ces composés sont exclus de la présente invention par les conditions (1) et (4) du disclaimer qui précisent que lorsque X représente (g), R₃ est différent du radical de formule (iii), (iv) ou (v) dans laquelle R₈ est un atome d'hydrogène, et lorsque X représente la liaison de formule (g) et R₃ représente le radical de formule (v), alors R₈ est différent d'un radical monohydroxyalkyle ou polyhydroxyalkyle, ou d'un radical cycloaliphatique en C3-C6.

Dans le documentEP-A-514264, il a été décrit des composés qui peuvent correspondre aux composés de formule (I) présentés ci-après dans lesquels X représente (h), (j) ou (k), R3 représente un atome d'hydrogène, un radical hydroxy, un radical alkoxy en C1-C6, un radical cycloalkyle ayant de 3 à 12 atomes de carbone, un radical alkényle ayant de 2 à 6 atomes de carbone, un radical mono ou polyhydryalkyle.
Ces composés sont exclus de la présente invention par les conditions (1) et (4) du disclaimer qui précisent que lorsque X représente (h), (j) ou (k), alors R₃ est différent du radical de formule (iii), (iv) ou (v) dans laquelle R₈ est un atome d'hydrogène, et lorsque X représente la liaison de formule (h), (j) ou (k) et R₃ représente le radical de formule (v), alors R₈ est différent d'un radical monohydroxyalkyle ou polyhydroxyalkyle, ou d'un radical cycloaliphatique en C3-C6.

Les composés décrits dans ces documents peuvent avoir les mêmes applications que celles des composés selon l'invention.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante : 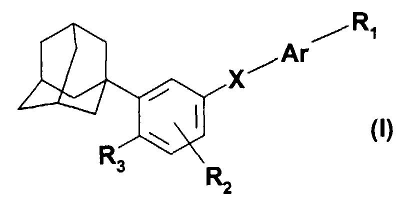 dans laquelle:
- R₁ représente
   (i) le radical -CH₃
   (ii) le radical -(CH₂)n-O-R₄
   (iii) le radical -O-(CH₂)m-(CO)n-R₅
   (iv) le radical -CO-R₆
   (v) le radical -CO-O-R₇
   les valeurs m et n, ainsi que les différents radicaux R₄ à R₇ ayant la signification donnée ci-après,
- R₂ représente un atome d'hydrogène, d'halogène, un radical alkyle linéaire ou ramifié ayant de 1 à 20 atomes de carbone, un radical -OR₄, un radical -O-CH₂-O-CH₂-CH₂-O-CH₃.
- R₃ représente:
   (i) un radical -Y-(CH₂)p-Y-(CH₂)q-R₈
   (ii) un radical -(CH₂)p-Y-(CH₂)q-R₈
   (iii) un radical -Y-(CH₂)q-R₈
   (iv) un radical -CH=CH-(CH₂)r-R₈
   (v) un radical -(CH₂)q-R₈
   les valeurs p, q, r et les radicaux Y et R₈ ayant la signification donnée ci-après,
- X représente les liaisons de formules (a)-(k) suivantes pouvant être lues dans les deux sens : 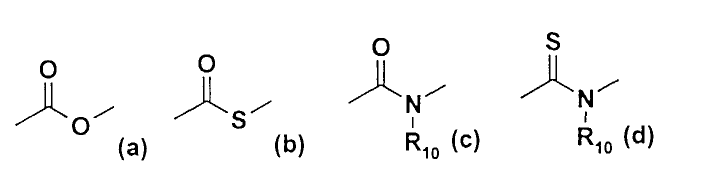 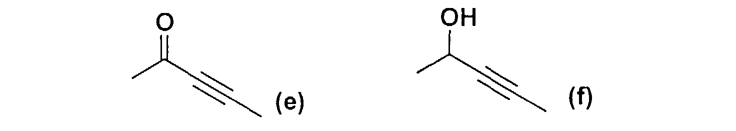 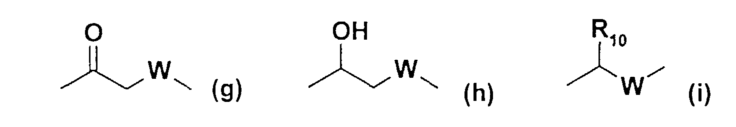 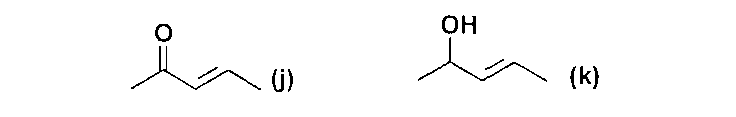
- Ar représente un radical choisi parmi les radicaux de formules (a)-(f) suivantes : 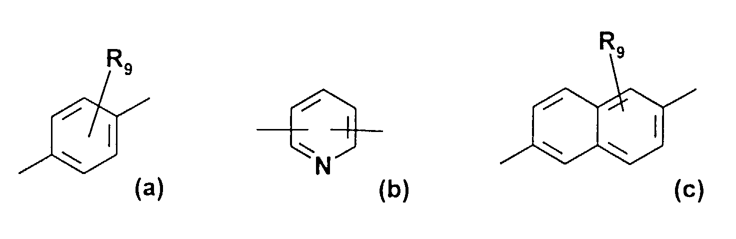 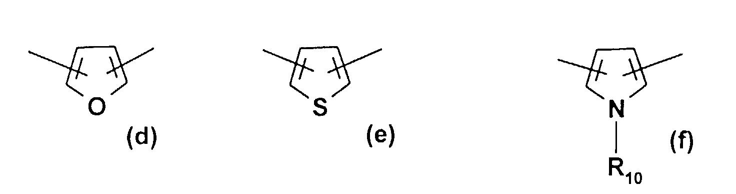 étant entendu que dans tout ce qui précède :
- m est un nombre entier égal à 1,2 ou 3
- n est un nombre entier égal à 0 ou 1
- p est un nombre entier compris inclusivement entre 1 et 12
- q est un nombre entier compris inclusivement entre 0 et 12
- r est un nombre entier compris inclusivement entre 0 et 10
- t est un nombre entier égal à 0, 1 ou 2
- Y représente l'atome d'oxygène ou le radical S(O)t
- W représente l'atome d'oxygène, le radical S(O)t ou le radical N-R₁₀
- R₄ représente un atome d'hydrogène, un radical alkyle inférieur ou un radical -CO-R₁₁
- R₅ représente un alkyle inférieur ou un hétérocycle
- R₆ représente un atome d'hydrogène, un radical alkyle inférieur ou un radical 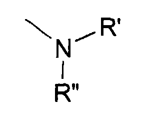 dans lequel R' et R" représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle.
- R₇ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide.
- R8 représente un atome d'hydrogène, un radical alkyle ramifié ayant de 1 à 20 atomes de carbone, un radical cycloaliphatique en C3-C6, un radical monohydroxyalkyle ou un radical polyhydroxyalkyle dont les hydroxy sont éventuellement protégés sous forme de méthoxy ou d'acétoxy ou d'acétonide, un radical aryle, un radical alkynyle, un radical -CO-R₆, un radical -CO-O-R₇, un radical aminoalkyle dont la fonction amine est éventuellement substituée par un ou deux groupements alkyles inférieurs, un hétérocycle, R₇ ayant la signification donnée ci-dessus
- R₉ représente un atome d'hydrogène, d'halogène, un radical allkyle linéaire ou ramifié ayant de 1 à 20 atomes de carbone, un radical -OR₄, un radical -O-CH₂-O-CH₂-CH₂-O-CH₃.
- R₁₀, identique ou différent, représente un atome d'hydrogène, un radical alkyle inférieur
- R₁₁ représente un radical alkyle inférieur
étant donné que lorsque:
- X représente la liaison de formule (a), (b), (c), (g), (h), (j) ou (k), alors R₃ est différent du radical de formule (iii), (iv) ou (v) dans laquelle R₈ est un atome d'hydrogène,
- X représente la liaison de formule (a), alors R₃ est différent du radical (iii),
- X représente la liaison de formule (b) et R₃ représente le radical de formule (iii) dans laquelle Y représente un atome de soufre, alors R₈ est différent du radical aryle,
- X représente la liaison de formule (g), (h), (j) ou (k) et R₃ représente le radical de formule (v), alors R₈ est différent d'un radical monohydroxyalkyle ou polyhydroxyalkyle, ou d'un radical cycloaliphatique en C3-C6.

L'invention vise également les sels des composés de formule (I) lorsque R₁ ou R₈ représente une fonction acide carboxylique et/ou lorsque R₈ représente une fonction amine, les analogues chiraux et les isomères géométriques desdits composés de formule (I). Lorsque les composés selon l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique. Lorsque les composés selon l'invention se présentent sous forme de sels par addition d'une base, il s'agit de sels d'un métal alcalin ou alcalino-terreux ou encore de zinc ou d'une amine organique.

Selon la présente invention, on entend par radical alkyle inférieur un radical ayant de 1 à 12, de préférence de 1 à 9, atomes de carbone, avantageusement les radicaux méthyle, éthyle, isopropyle, butyle, tertiobutyle, hexyle, nonyle et dodécyle.

Par radical alkyle linéaire ayant de 1 à 20 atomes de carbone, on entend notamment les radicaux méthyle, éthyle, propyle, 2-éthyl-hexyle, octyle, dodécyle, hexadécyle et octadécyl.

Par radical alkyle ramifié ayant de 1 à 20 atomes de carbone, on entend notamment les radicaux 2-méthylbutyle, 2-méthylpentyle, 1-méthylhexyle, 3-méthylheptyle.

Parmi les radicaux monohydroxyalkyle, on préfère un radical présentant 2 ou 3 atomes de carbone, notamment un radical 2-hydroxyéthyle, 2-hydroxypropyle ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on préfère un radical phényle éventuellement substitué par au moins un atome d'halogène, un hydroxyle ou une fonction nitro.

Parmi les radicaux aralkyle, on préfère le radical benzyle ou phénéthyle éventuellement substitué par au moins un atome d'halogène, un hydroxyle ou une fonction nitro.

Parmi les radicaux alkényle, on préfère un radical contenant de 1 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Par radical aminoalkyle, on entend un radical contenant de préférence de 1 à 6 atomes de carbone, notamment les radicaux aminométhyle, 3-aminopropyle, 6-aminohexyle.

Parmi les radicaux alkynyle, on préfère un radical ayant de 2 à 6 atomes de carbone, notamment un radical propargyle.

Parmi les radicaux cycloaliphatiques de 3 à 6 atomes de carbone, on peut citer plus particulièrement le radical cyclopropyle et cyclohexyle.

Lorsque les radicaux R₂ et R₉ représentent un atome d'halogène, celui-ci est de préférence un atome de fluor, de brome ou de chlore.

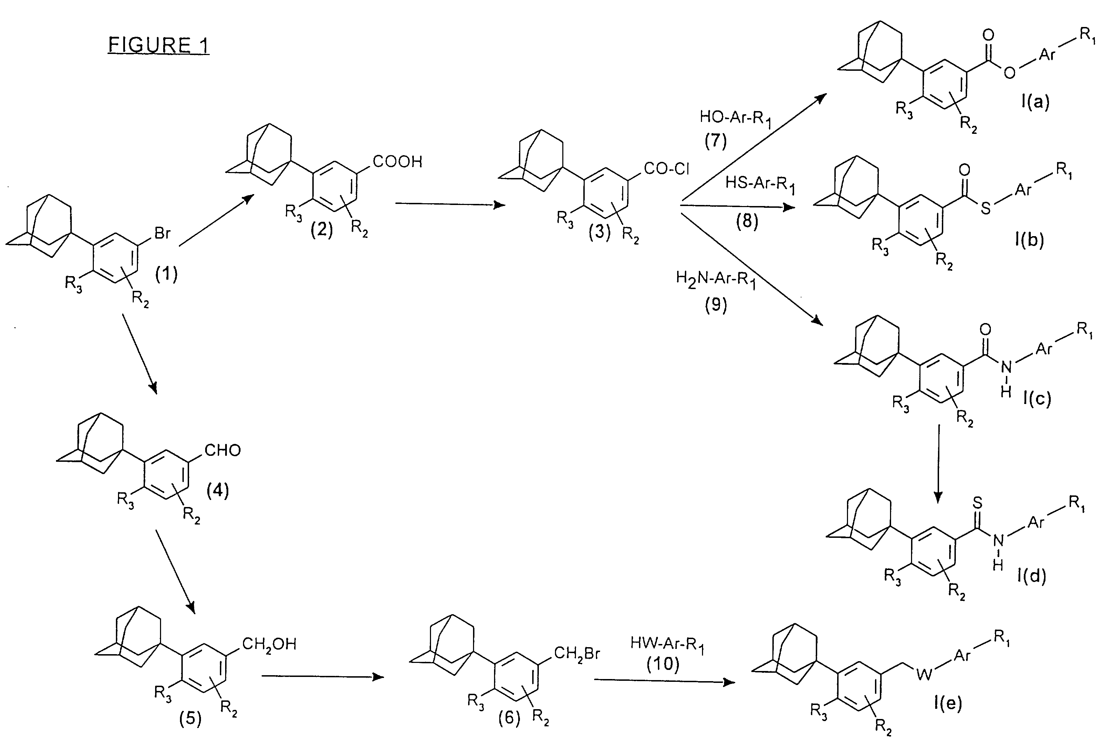

Parmi les composés de formule (I) ci-dessus rentrant dans le cadre de la présente invention, on peut notamment citer les suivants:
Acide 4-[3-(1-adamantyl)-4-methoxyphénylthiométhyl]benzoïque.
Acide 4-[3-(1-adamantyl)-4-methoxyphénylsulfinylméthyl]benzoïque.
Acide 4-[3-(1-adamantyl)-4-methoxyphénylméthylsulfonyl]benzoïque.
Acide 4-[3-(1-adamantyl)-4-methoxyphénylmèthylsulfinyl]benzoïque.
Acide 4-[3-(1-adamantyl)4-methoxyphénylsulfonylméthyl]benzoïque.
Acide 4-[3-(1-adamantyl)-4-methoxyphénylthiocarboxamido]benzoïque.
Acide 4-[3-(1-adamantyl)-4-methoxyphénylméthylthio]benzoïque.
Acide 4-[3-(1-adamantyl)-4-methoxyphénylméthylamino]benzoïque.
Acide 4-[[3-oxo-3-[3-(1-adamantyl)-4-methoxyphényl]-1-propynyl]]benzoïque.
Acide 4-[3-(1-adamantyl)4-methoxyethoxymethoxybenzamido]benzoïque.
Acide 4-[3-(1-adamantyl)-4-methoxyethoxymethoxybenzoyloxy]benzoïque.
Acide (E)-4-[[3-oxo-3-[3-(1-adamantyl)-4-methoxyethoxymethoxyphenyl]-1-propenyl]]benzoïque.
Acide 4-[[3-hydroxy-3-[3-(1-adamantyl)-4-methoxyethoxymethoxyphenyl]-1-propynyl]]benzoïque.
Acide 4-[3-(1-adamantyl)-4-(2,2-diméthyl-1,3-dioxolanne-4-méthyloxy) phenylcarboxamido]benzoïque.
Acide 4-[3-(1-adamantyl)-4-(2,3-dihydroxypropyloxy)phenylcarboxamido] benzoïque.
Acide 4-[3-(1-adamantyl)-4-méthoxycarbonylméthyloxyphénylcarboxamido] benzoïque..
Acide 2-[3-(1-adamantyl)-4-méthoxyphénoxyméthyl]-4-thiophenecarboxylique.
Acide 2-[3-(1-adamantyl)4-méthoxyphénylaminométhyl]-4-thiophene carboxylique.
Acide 2-[3-(1-adamantyl)4-méthoxyphénylthiométhyl]-4-thiophene carboxylique.
Acide 4-[3-(1-adamantyl)-4-(2,2-diméthyl-1,3-dioxolanne-4-méthyloxy) benzoyloxy]benzoïque.
Acide 4-[3-hydroxy-3-[5-(1-adamantyl)-2,4-dimethoxyethoxymethoxyphenyl]-1-propynyl]benzoïque.
Acide 4-[3-(1-adamantyl)-4-methoxyethoxymethoxybenzoylthio]benzoïque.
Acide N-methyl-4-[3-(1-adamantyl)-4-methoxyethoxymethoxyphenyl carboxamido]benzoïque.
Acide -4-[3-(1-adamantyl)-4-methoxyethoxymethoxyphenylthiocarboxamido] benzoïque.
Acide (E)-4-[[3-oxo-3-[3-(1-adamantyl)-4-methoxymethoxyphenyl]-1-propenyl]]benzoïque.
Acide (E)-4-[[3-oxo-3-[3-(1-adamantyl)4-hydroxyphenyl]-1-propenyl]] benzoïque.
Acide (E)-4-[[3-oxo-3-[3-(1-adamantyl)-4-methoxyphenyl]-1-propenyl]] benzoïque.
Acide (E)-4-[[3-oxo-3-[3-(1-adamantyl)-4-(3-hydroxypropyloxy)phenyl]-1-propenyl]]benzoïque.
4-[3-(1-adamantyl)-4-methoxyethoxymethoxybenzoyloxy]benzaldéhyde.
4-[3-(1-adamantyl)-4-methoxyethoxymethoxybenzoyloxy]benzeneméthanol.
(E)-N-éthyl-4-[[3-oxo-3-[3-(1-adamantyl)-4-methoxyethoxymethoxyphenyl]-1-propenyl]]benzamide.
(E)-N-(4-hydroxyphenyl)-4-[[3-oxo-3-[3-(1-adamantyl)-4-methoxyethoxy methoxyphenyl]-1-propenyl]]benzamide.
(E)-4-[[3-oxo-3-[3-(1-adamantyl)-4-methoxyethoxymethoxyphenyl]-1-propenyl]]phenol.
Selon la présente invention les composés de formule (I) plus particulièrement préférés sont ceux pour lesquels au moins l'une des, et de préférence toutes les, conditions suivantes est remplie :
- R₁ est le radical
   -COO-R₇ ou -CO-R₆.
- R₃ est le radical
   Y-(CH₂)p-Y-(CH₂)q-R₈.
   -CH₂)p-Y-(CH₂)q-R₈.
   -Y-(CH₂)q-R₈.
- X représente une liaison de formule (a), (e), (f), (j) ou (k).
- Ar représente le radical de formule (a) ou (b).
La présente invention a également pour objet les procédés de préparation des composés de formule (I), en particulier selon les schémas réactionnels donnés aux figures 1, 2 et 3.

Ainsi les composés de formule l(a) peuvent être obtenus (figure 1) en faisant réagir en milieu anhydre, dans un solvant organique de préférence le THF et en présence d'une amine tertiaire (par exemple triéthylamine ou pyridine) une forme activée d'un acide benzoïque (2) par exemple un chlorure d'acide (3) sur un composé phénolique de formule (7).

Ainsi les composés de formule l(b) peuvent être obtenus (figure 1) en faisant réagir en milieu anhydre, dans un solvant organique de préférence le THF et en présence d'une amine tertiaire (par exemple triéthylamine ou pyridine) une forme activée d'un acide benzoïque (2) par exemple un chlorure d'acide (3) sur un composé thiophénolique de formule (8).

Ainsi les composés de formule l(c) peuvent être obtenus (figure 1) en faisant réagir en milieu anhydre, dans un solvant organique de préférence le THF et en présence d'une amine tertiaire (par exemple triéthylamine ou pyridine) une forme activée d'un acide carboxylique aromatique (2) par exemple un chlorure d'acide (3) sur un composé aminé de formule (9).

Ainsi les composés de formule l(d) peuvent être obtenus (figure 1) à partir des composés de formule 1(c) par réaction avec le réactif de Lawesson.

Ainsi les composés de formule 1(e) peuvent être préparés (figure 1) à partir des alcools benzyliques (5) par transformation en dérivés bromés (6) avec du tribromure de phosphore puis réaction en présence de carbonate de potassium ou d'un hydrure alcalin (hydrure de sodium) ou par transfert de phase en utilisant par exemple le bromure de tétrabutylammonium comme sel quaternaire avec un composé (10) porteur d'une fonction hydroxy ou thiol ou amino.

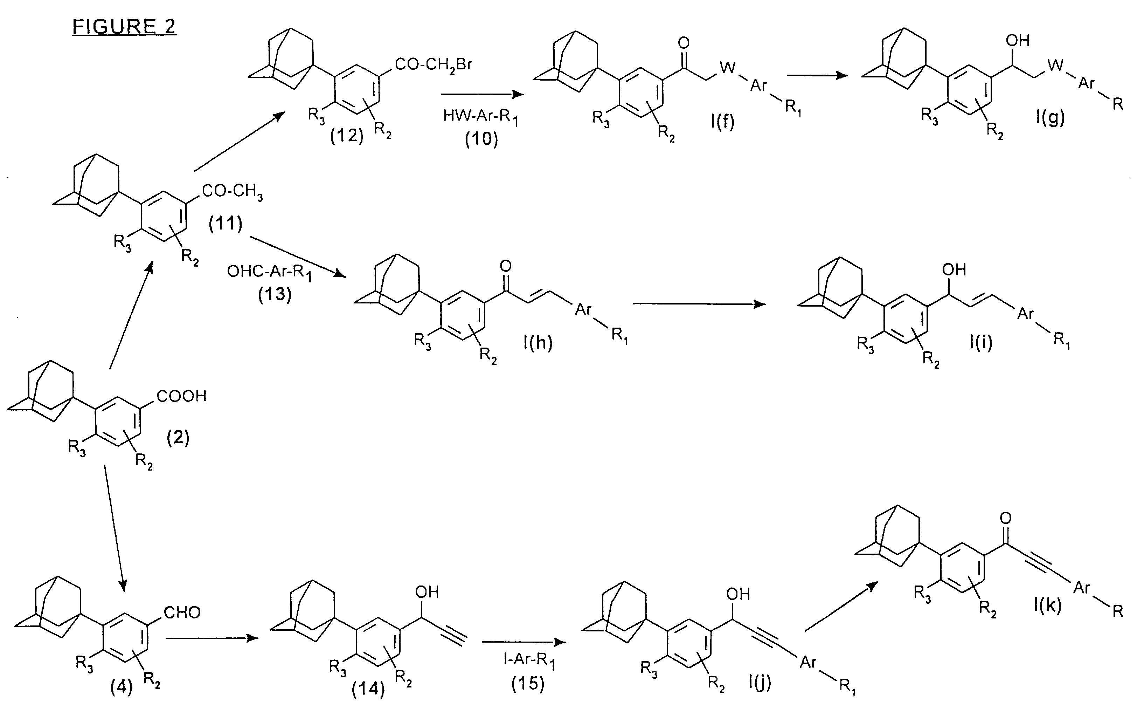

Ainsi les composés de formule l(f) peuvent être obtenus (figure 2) à partir des dérivés acetophenones (11) par transformation en dérivé bromé (12) à l'aide du brome puis réaction en présence de carbonate de potassium ou d'un hydrure alcalin (hydrure de sodium) ou par transfert de phase en utilisant par exemple le bromure de tétrabutylammonium comme sel quaternaire avec un composé (10) porteur d'une fonction hydroxy ou thiol ou amino. Les composés de formule l(g) peuvent être obtenus à partir du dérivé l(f) par réaction avec du borohydrure de sodium dans un solvant alcoolique.

Ainsi les composés de formule l(h) peuvent être obtenus (figure 2) à partir des dérivés acetophenones (11) par réaction avec des dérives aldéhydes aromatiques (13) en présence de méthylate de sodium ou de soude dans un solvant alcoolique tel le méthanol. A partir de ces composés par réaction avec du borohydrure de sodium en présence de trichlorure de Cerium, on obtient les composés de formule l(i).

Ainsi les composés de formule l(j) peuvent être préparés (figure 2) à partir des dérives aldéhydes aromatiques (4) par réaction avec le triméthylsilylacétylénure de lithium puis déprotection avec le fluorure de tétrabutylammonium pour obtenir les dérivés a-hydroxyacétyléniques (14). Puis couplage avec les dérivés halogénés (15) de préférence iodé en présence d'un catalyseur au Palladium (par exemple le chlorure de Bis-(triphénylphosphine)palladium(ll) dans un solvant tel la triéthylamine. L'oxydation de ces composés avec soit le pyridinium dichromate ou l'oxyde de manganèse ou le réactif de Swern conduit aux dérivés de formule l(k).

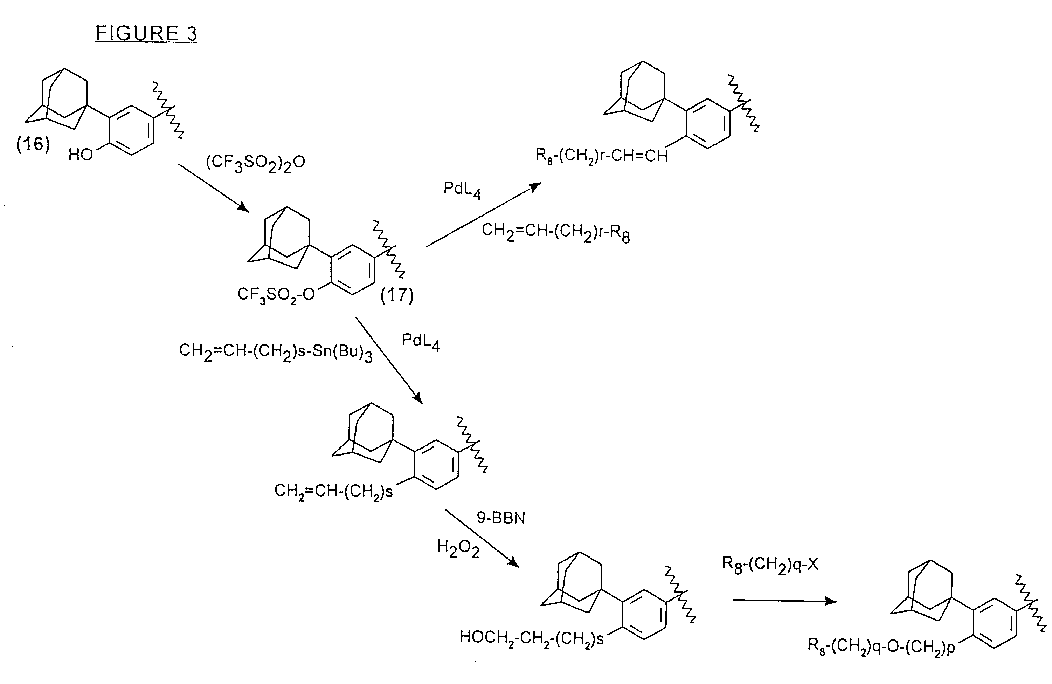

Lorsque R₃ représente les radicaux -(CH2)p-Y-(CH2)q-R₈ ou -CH=CH-(CH2)r-R₈, les composés peuvent être obtenus (figure 3 dans laquelle s égal p-2) à partir des dérivés phénoliques (16) que l'on transforme en dérivés triflates (17) puis substitution nucléophile en présence d'un catalyseur au palladium selon les conditions générales décrites par:
- S. Cacchi et al. Tetrahedron Letter, 1986, 27, 3931-3934.
- W. J. Scott et al. J. Org. Chem. 1985, 50, 2302-2308.
- J. K. Stille et al. J. Am. Chem. Soc; 1987, 109, 5478-5486.

Lorsque R₁ représente le radical -COOH, les composés sont préparés en protégeant R₁ par un groupe protecteur de type alkyle, allylique, benzylique ou tert-butylique.

Le passage à la forme libre peut être éffectué:
- dans le cas d'un groupe protecteur alkyle, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupement protecteur benzylique, par débenzylation en présence d'hydrogène au moyen d'un catalyseur tel que le palladium sur charbon.
- dans le cas d'un groupement protecteur de type tert-butylique au moyen d'iodure de triméthylsilyle.

La présente invention a également pour objet à titre de médicament les composés de formule (I) telle que définie ci-dessus.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités de ces composés respectivement dans les domaines de la différenciation et de la prolifération cellulaire. Dans le test de différenciation des cellules (F9), il est possible d'évaluer une activité agoniste, comme une activité antagoniste aux récepteurs de l'acide rétinoïque. Un antagoniste est inactif lorsqu'il est seul dans ce test, mais inhibe partiellement ou totalement l'effet produit par un rétinoïde agoniste sur la morphologie et sur la sécrétion de l'activateur plasminogène. Certains de ces composés présentent donc également une activité dans un test qui consiste à identifier des molécules antagonistes des RARs, tel que décrit dans la demande de brevet français n° 95-07302 déposée le 19 juin 1995 par la Demanderesse. Ce test comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être inhibée par l'administration par voie systémique ou topique d'une molécule antagoniste des RARs.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des a-hydroxy ou a-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par a-hydroxy ou a-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des a-hydroxy ou a-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphénylimidazolidine 2,4-dione); des agents anti-inflammatoires non stéroïdiens; des caroténoïdes et, notamment, le b-carotène; des agents anti-psoriatiques tels que l'anthraline et ses dérivés; et enfin les acides eicosa-5,8,11,14-tétraynoïque et eicosa-5,8,11-triynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### Acide 4-[3-(1-adamantyl)-4-methoxyphénylthiométhyl]benzoïque.

### (a) O-3-(1-adamantyl)-4-méthoxyphényldimethylthiocarbamate.

Dans un ballon et sous courant d'azote, on introduit 600 mg (20 mmoles) d'hydrure de sodium (80% dans l'huile) et 50 ml de DMF. On refroidit à 0°C et ajoute goutte à goutte une solution de 5,5 g (20 mmoles) de 3-(1-adamantyl)-4-méthoxy phénol dans 100 ml de DMF et agite jusqu'à cessation du dégagement gazeux, on ajoute ensuite une solution de 3,3 g (26 mmoles) de chlorure de dimethylthiocarbamoyle dans 50 ml de DMF et agite pendant huit heures à température ambiante. On verse le milieu réactionnel dans l'eau, extrait avec de l'acétate d'éthyle, décante la phase organique, lave à l'eau, sèche sur sulfate de magnésium, évapore. Le résidu obtenu est purifié par chromatographie sur colonne de silice élué avec un mélange de dichlorométhane et d'hexane (70-30). Après évaporation des solvants, on recueille 3,1 g (46%) du produit attendu de point de fusion 162-4°C.

### (b) S-3-(1-adamantyl)-4-méthoxyphényldimethylthiocarbamate.

Dans un ballon et sous courant d'azote, on introduit 3 g (8,7 mmoles) du produit précédent et chauffe à 300°C pendant trente minutes. On extrait le milieu réactionnel avec du dichloromethane, lave à l'eau, décante la phase organique, sèche sur sulfate de magnésium, évapore. On recueille 2,4 g (80%) du produit attendu de point de fusion 154-5°C.

### (c) 3-(1-adamantyl)-4-méthoxyphénylthiol.

Dans un ballon, on introduit 2,3 g (6,6 mmoles) du produit précédent et 50 ml d'une solution de soude méthanolique (2N) et chauffe à reflux pendant trois heures. On évapore le milieu réactionnel, reprend par l'eau, acidifie avec de l'acide chlorhydrique concentré, filtre. Le résidu obtenu est purifié par chromatographie sur colonne de silice élué avec un mélange de dichlorométhane et d'hexane (10-90). Après évaporation des solvants, on recueille 1,2 g (66%)) du dérivé thiol attendu de point de fusion 149-51°C.

### (d) Acide 4-[3-(1-adamantyl)-4-methoxyphénylthiométhyl]benzoïque.

Dans un tricol et sous courant d'azote, on introduit 1,1 g (36,6 mmoles) d'hydrure de sodium (80% dans l'huile) et 50 ml de DMF. On ajoute goutte à goutte une solution de 4,2 g (15,2 mmoles) de 3-(1-adamantyl)-4-méthoxyphénylthiol dans 20 ml de DMF et agite jusqu'à cessation du dégagement gazeux. On ajoute ensuite une solution de 3,3 g (15,2 mmoles) d'acide 4-bromométhylbenzoïque dans 20 ml de DMF et agite à température ambiante pendant huit heures. On verse le milieu réactionnel dans l'eau, acidifie à pH 1 avec de l'acide chlorhydrique, extrait avec de l'éther éthylique, décante la phase organique, lave à l'eau, sèche sur sulfate de magnésium, évapore. Le résidu obtenu est purifié par chromatographie sur colonne de silice élué avec un mélange de dichlorométhane et d'éther éthylique (95-5). Après évaporation des solvants, on recueille 4,2 g (68%) du produit attendu de point de fusion 204-5°C.

### EXEMPLE 2

### Acide 4-[3-(1-adamantyl)-4-methoxyphénylsulfinylméthyl]benzoïque.

### (a) 4-[3-(1-adamantyl)-4-methoxyphénylthiométhyl]benzoate de méthyle.

Dans un ballon, on introduit 5 g (12,3 mmoles) d'acide 4-[3-(1-adamantyl)-4-methoxyphénylthiométhyl]benzoïque 50 ml de méthanol et ajoute 330 µl d'acide sulfurique concentré. On chauffe à reflux pendant huit heures puis évapore le milieu réactionnel à sec. On reprend par de l'eau, neutralise avec du bicarbonate de sodium, extrait avec de l'éther éthylique, décante la phase organique, sèche sur sulfate de magnésium, évapore. Le résidu obtenu est purifié par chromatographie sur colonne de silice élué avec un mélange de dichlorométhane et de l'hexane (40-60). Après évaporation des solvants, on recueille 4,1 g (79%) de 4-[3-(1-adamantyl)-4-methoxyphénylthiométhyl]benzoate de méthyle.

### (b) 4-[3-(1-adamantyl)-4-methoxyphénylsulfinylméthyl]benzoate de méthyle.

Dans un ballon on introduit 844 mg (2 mmoles) de 4-[3-(1-adamantyl)-4-methoxyphénylthiométhyl]benzoate de méthyle 20 ml de dichlorométhane et ajoute 386 mg (1,9 mmoles) d'acide méta-chloroperbenzoïque. On agite à température ambiante pendant deux heures, verse le milieu réactionnel dans l'eau, extrait avec du dichlorométhane, décante la phase organique, sèche sur sulfate de magnésium, évapore. Le résidu est purifié par chromatographie sur colonne de silice élué avec un mélange de dichlorométhane et d'éther éthylique (90-10). Après évaporation des solvants, on recueille 270 mg (30%) de l'ester attendu de point de fusion 121-2°C.

### (c) Acide 4-[3-(1-adamantyl)-4-methoxyphénylsulfinylméthyl]benzoïque.

Dans un ballon, on introduit 1,1 g (2,7 mmoles) de l'ester méthylique précédent, 40 ml de THF et 40 ml d'une solution de soude méthanolique (2N) et agite à température ambiante pendant huit heures. On évapore à sec le milieu réactionnel, reprend par l'eau, acidifie à pH 1, extrait avec de l'éther éthylique, décante la phase organique, sèche sur sulfate de magnèsium, évapore. Le résidu obtenu est trituré dans l'hexane, filtré et séché. On recueille 900 mg (78%) de l'acide attendu de point de fusion 164-5°C.

### EXEMPLE 3

### Acide 4-[3-(1-adamantyl)-4-methoxyphénylméthylsulfonyl]benzoïque.

### (a) 4-[3-(1-adamantyl)-4-methoxyphénylméthylsulfonyl]benzoate de méthyle.

De manière analogue à l'exemple 2(b) par réaction de 4,22 g (10 mmoles) de 4-[3-(1-adamantyl)-4-methoxyphénylméthylthio]benzoate de méthyle avec 10, 35 g (30 mmoles) d'acide méta-chloroperbenzoïque, on obtient 1,66 g (37%) de l'ester méthylique attendu de point de fusion 168-70°C.

### (b) acide 4-[3-(1-adamantyl)-4-methoxyphènylméthylsulfonyl]benzoïque.

De manière analogue à l'exemple 2(c) à partir de 3,98 g (8,7 mmoles) de l'ester méthylique précédent, on obtient 3,13 g (81%) de l'acide attendu de point de fusion 231-2°C.

### EXEMPLE 4

### Acide 4-[3-(1-adamantyl)-4-methoxyphénylméthylsulfinyl]benzoïque.

### (a) 4-[3-(1-adamantyl)4-methoxyphénylméthylsulfinyl]benzoate de méthyle.

De manière analogue à l'exemple 2(b) par réaction de 2,96 g (7 mmoles) de 4-[3-(1-adamantyl)-4-methoxyphénylméthylthio]benzoate de méthyle avec 1,42 g (7 mmoles) d'acide méta-chloroperbenzoïque, on obtient 2,33 g (76%) de l'ester méthylique attendu de point de fusion 151-2°C.

### (b) acide 4-[3-(1-adamantyl)-4-methoxyphénylméthylsulfinyl]benzoïque.

De manière analogue à l'exemple 2(c) à partir de 2,7 g (6,2 mmoles) de l'ester méthylique précédent, on obtient 2,36 g (90%) de l'acide attendu de point de fusion 192-3°C.

### EXEMPLE 5

### Acide 4-[3-(1-adamantyl)-4-methoxyphénylsulfonylméthyl]benzoïque.

### (a) 4-[3-(1-adamantyl)-4-methoxyphénylsulfonylméthyl]benzoate de méthyle.

Dans un ballon on introduit 1,8 g (4,2 mmoles) de 4-[3-(1-adamantyl)-4-methoxyphénylthiométhyl]benzoate de méthyle 20 ml de dichlorométhane et ajoute 3,4 g (16,9 mmoles) d'acide méta-chloroperbenzoïque. On agite à température ambiante pendant deux heures, verse le milieu réactionnel dans l'eau, extrait avec du dichlorométhane, décante la phase organique, sèche sur sulfate de magnésium, évapore. Le résidu est purifié par chromatographie sur colonne de silice élué avec un mélange de dichlorométhane et d'hexane (90-10). Après évaporation des solvants, on recueille 1,8 g (93%) de l'ester attendu de point de fusion 166-8°C.

### (b) Acide 4-[3-(1-adamantyl)-4-methoxyphénylsulfonylméthyl]benzoïque.

De manière analogue à l'exemple 2(a) à partir de 2,1 g (4 mmoles) de l'ester méthylique précédent, on obtient 1,3 g (68%) de l'acide attendu de point de fusion 238-9°C.

### EXEMPLE 6

### Acide 4-[3-(1-adamantyl)-4-methoxyphénylthiocarboxamido]benzoïque.

### (a) 4-[3-(1-adamantyl)-4-methoxyphénylthiocarboxamido]benzoate de méthyle.

Dans un ballon, on introduit 6,7 g (16,6 mmoles) de 4-[3-(1-adamantyl)-4-methoxyphénylcarboxamido]benzoate de méthyle 60 ml de toluène et ajoute 4,1 g (9,9 mmoles) de réactif de Lawesson. On chauffe à reflux pendant trois heures, évapore le milieu réactionnel à sec. On reprend le résidu obtenu par l'eau et le dichlorométhane, décante la phase organique, sèche sur sulfate de magnésium, évapore. Le résidu obtenu est purifié par chromatographie sur colonne de silice élué avec du dichlorométhane. Après évaporation des solvants, on recueille 6 g (83%) de l'ester attendu de point de fusion 198-200°C.

### (b) acide 4-[3-(1-adamantyl)-4-methoxyphénylthiocarboxamido]benzoïque.

De manière analogue à l'exemple 2(a) à partir de 6 g (14,3 mmoles) de l'ester méthylique précédent, on obtient 5,6 g (92%) de l'acide attendu de point de fusion 255-6°C.

### EXEMPLE 7

### Acide 4-[3-(1-adamantyl)-4-methoxyphénylméthylthio]benzoïque.

### (a) 3-(1-adamantyl)-4-methoxybenzèneméthanol.

Dans un tricol et sous courant d'azote, on introduit 8 g (0,2 mole) d'hydrure double de lithium et d'aluminium et 50 ml de THF anhydre. On ajoute goutte à goutte une solution de 28,6 g (0,1 mole) d'acide 3-(1-adamantyl)-4-methoxybenzoïque dans 260 ml de THF anhydre et chauffe à reflux pendant 16 heures. On refroidit le milieu réactionnel et hydrolyse avec 14,4 ml d'une solution de tartrate double de sodium et de potassium. On filtre le sel, évapore le filtrat, triture le solide obtenu dans l'hexane, filtre, sèche. On recueille 26,2 g (96%) de l'alcool benzylique attendu de point de fusion 134-5°C.

### (b) bromure de 3-(1-adamantyl)-4-méthoxybenzyle.

Dans un tricol, on introduit 2,72 g (10 mmoles) de l'alcool benzylique précédent 30 ml de toluène et 800 µl (10 mmoles) de pyridine. A 0°C, on ajoute goutte à goutte une solution de 940 µl (10 mmoles) de tribromure de phosphore dans 9 ml de toluène et agite à température ambiante 4 heures. On évapore le milieu réactionnel à sec, reprend le résidu par l'eau et l'éther éthylique, décante la phase organique, sèche sur sulfate de magnésium, évapore. Le résidu obtenu est trituré dans l'hexane, filtré et sèché. On recueille 2,35 g (70%) du bromure de benzyle attendu de point de fusion 95-9°C.

### (c) 4-[3-(1-adamantyl)-4-methoxyphénylméthylthio]benzoate de méthyle.

Dans un tricol et sous courant d'azote, on introduit 1,44 g (48 mmoles) d'hydrure de sodium (80% dans l'huile) et 25 ml de DMF. On ajoute goutte à goutte une solution de 6,73 g (40 mmoles) de 4-mercaptobenzoate de méthyle dans 60 ml de DMF et agite jusqu'à cessation du dégagement gazeux. On ajoute ensuite une solution de 16,1 g (48 mmoles) de bromure de 3-(1-adamantyl)-4-méthoxybenzyle dans 90 ml de DMF et agite à température ambiante pendant huit heures. On verse le milieu réactionnel dans l'eau,acidifie à pH 1 avec de l'acide chlorhydrique extrait avec de l'éther éthylique, décante la phase organique, lave à l'eau, sèche sur sulfate de magnésium, évapore. Le résidu obtenu est purifié par chromatographie sur colonne de silice élué avec un mélange de dichlorométhane et d'hexane (40-60). Après évaporation des solvants, on recueille 16,75 g (99%) de l'ester méthylque attendu de point de fusion 143-5°C.

### (d) acide 4-[3-(1-adamantyl)-4-methoxyphénylméthylthio]benzoïque.

De manière analogue à l'exemple 2(a) à partir de 4,22 g (10 mmoles) de l'ester méthylique précédent, on obtient 3,15 g (77%) de l'acide attendu de point de fusion 225-7°C.

### EXEMPLE 8

### Acide 4-[3-(1-adamantyl)-4-methoxyphénylméthylamino]benzoïque.

### (a) N-tert-butyloxycarbonyl-3-(1-adamantyl)-4-methoxyphénylméthylamino]benzoate de benzyle.

De manière analogue à l'exemple 7(c) par réaction de 10 6 g (34,6 mmoles) de bromure de 3-(1-adamantyl)-4-méthoxybenzyle avec 8,6 g (26,3 mmoles) de 4-tert-butyloxycarboxamidobenzoate de benzyle, on obtient 14 g (91%) de l'ester benzylique attendu sous forme d'une huile jaune.

### (b) 3-(1-adamantyl)-4-methoxyphénylméthylamino]benzoate de benzyle.

Dans un tricol et sous courant d'azote, on introduit 13,96 g (24 mmoles) de l'ester benzylique précédent et 100 ml de tétrachlorure de carbone. On ajoute goutte à goutte 5 ml (48 mmoles) d'iodure de triméthylsylane et agite à température ambiante pendant 12 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (55-45). Après évaporation des solvants, on recueille 6,64 g (57%) de l'ester benzylique attendu.

### (c) acide 4-[3-(1-adamantyl)-4-methoxyphénylméthylamino]benzoïque.

De manière analogue à l'exemple 2(a) à partir de 1,44 g (3 mmoles) de l'ester benzylique précédent, on obtient 877 mg (75%) de l'acide attendu de point de fusion 259-60°C.

### EXEMPLE 9

### Acide 4-[[3-oxo-3-[3-(1-adamantyl)-4-methoxyphényl]-1-propynyl]]benzoïque.

### (a) 4-triméthylsilyléthynylbenzoate de méthyle.

Dans un tricol et sous courant d'azote , on introduit 21,5 g (0,1 mole) de 4-bromobenzoate de méthyle, 300 ml de triéthylamine et un mélange de 200 mg d'acétate de palladium et de 400 mg de triphénylphosphine. On ajoute ensuite 20 g (0,204 mole)de triméthylsilylacétylène, chauffe progressivement à 90°C durant 1 heure et laisse à cette température pendant 5 heures. On refroidit le milieu réactionnel, filtre le sel et évapore le filtrat. On reprend le résidu avec 200 ml d'acide chlorhydrique (5%) et 400 ml d'éther éthylique. On décante la phase éthérée, lave à l'eau, sèche sur sulfate de magnésium, évapore. Le résidu obtenu est purifié par chromatographie sur colonne de silice, élué avec du dichlorométhane. Après évaporation des solvants, on recueille 23 g (100%) du dérivé attendu sous forme d'une huile incolore.

### (b) 4-[[3-oxo-3-[3-(1-adamantyl)-4-methoxyphényl]-1-propynyl]]benzoate de méthyle.

Dans un ballon, on introduit 3 g (10 mmoles) de chlorure de 3-(1-adamantyl)-4-methoxybenzoyle 2,3 g (10 mmoles) du dérivé précédent et 100 ml de dichlorométhane. On ajoute à 0°C par petites quantités 4,7 g (35 mmoles) d'AlCl₃ et agite à température ambiante pendant 8 heures. On verse le milieu réactionnel dans la glace, extrait avec du dichlorométhane, décante la phase organique, sèche sur sulfate de magnésium, évapore. Le résidu obtenu est purifié par chromatographie sur colonne de silice élué avec un mélange de dichlorométhane et d'hexane (80-20). On recueille 970 mg (23%) de produit attendu, de point de fusion 156-8°C.

### (c) acide 4-[[3-oxo-3-[3-(1-adamantyl)-4-methoxyphényl]-1-propynyl]]benzoïque.

Dans un ballon, on introduit 962 mg (2,2 mmoles) de l'ester méthylique précédent 10 ml de THF et ajoute 283 mg (6,7 mmoles) d'hydroxyde de lithium. On chauffe à reflux pendant 5 heures, verse le milieu réactionnel dans l'eau, acidifie à pH 1 avec de l'acide chlorhydrique concentré, décante la phase organique, sèche sur sulfate de magnésium, évapore. Le solide obtenu est trituré dans l'hexane, filtré, sèché. On recueille 600 mg (85%) de l'acide attendu de point de fusion 232-4°C.

### EXEMPLE 10

### Acide 4-[3-(1-adamantyl)-4-methoxyethoxymethoxybenzamido]benzoïque.

### (a) 3-(1-adamantyl)-1-bromo-4-méthoxyéthoxyméthoxybenzène.

Dans un tricol et sous courant d'azote on introduit 3,8 g (0,13 mole) d'hydrure de sodium (80% dans l'huile) 50 ml de DMF, ajoute goutte à goutte une solution de 40 g (0,13 mole) de 2-(1-adamantyl)-4-bromophénol dans 100 ml de DMF et agite jusqu'a cessation du dégagement gazeux. On ajoute ensuite goutte à goutte une solution de 18 ml (0,15 mole) de chlorure de 2-méthoxyéthoxyméthyle dans 20 ml de DMF et agite pendant quatre heures à température ambiante. On verse le milieu réactionnel dans l'eau, extrait avec de l'éther éthylique, décante la phase organique, lave à l'eau, sèche sur sulfate de magnésium, évapore. Le résidu est purifié par chromatographie sur colonne de silice élué avec un mélange de dichlorométhane et d'hexane (50-50). Après évaporation des solvants, on recueille 40,1 g (78%) du produit attendu de point de fusion 69-70°C.

### (b) acide 3-(1-adamantyl)-4-méthoxyéthoxyméthoxybenzoïque.

Le composé précédent (28,5 g, 72 mmoles) est dissous dans 200 ml de THF. La solution obtenue est ajoutée goutte à goutte sur du magnésium (2,4 g, 100 mmoles) et un cristal d'iode. Après introduction, on chauffe à reflux pendant deux heures, refroidit à -78°C et fait passer un courant de CO₂ pendant une heure. On laisse remonter à température ambiante, verse le milieu réactionnel dans une solution aqueuse saturée en chlorure d'ammonium, extrait avec de l'éther éthylique, décante la phase organique, sèche sur sulfate de magnésium, évapore. Le résidu obtenu est trituré dans l'hexane, filtré et séché. On recueille 15,5 g (60%) de l'acide attendu de point de fusion 115-6°C.

### (c) chlorure de 3-(1-adamantyl)-4-méthoxyéthoxyméthoxybenzoyle.

Dans un ballon, on introduit une solution de 3 g (8,3 mmoles) d'acide 3-(1-adamantyl)-4-méthoxyéthoxyméthoxybenzoïque dans 50 ml de dichlorométhane anhydre ajoute 1,7 ml (8,3 mmoles) de dicyclohexylamine et agite pendant une heure. On ajoute ensuite 600 µl (8,3 mmoles) de chlorure de thionyle et agite une heure. On évapore à sec, reprend par l'éther éthylique anhydre, filtre le sel de dicyclohexylamine et évapore le filtrat. On recueille 3,3 g (100%) du chlorure d'acide brut qui sera utilisé tel quel pour la suite de la synthèse.

### (d) 4-[3-(1-adamantyl)-4-methoxyethoxymethoxybenzamido]benzoate d'allyle.

Dans un ballon, on introduit 700 mg (3,95 mmoles) de 4-aminobenzoate d'allyle 1 ml (4,3 mmoles) de triéthylamine et 50 ml de THF. On ajoute goutte à goutte une solution de 1,5 g (3,96 mmoles) de chlorure de 3-(1-adamantyl)-4-méthoxyéthoxyméthoxybenzoyle et agite à température ambiante pendant 4 heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec du dichlorométhane. On recueille 1,7 g (83%) de l'ester allylique attendu sous forme d'une huile.

### (e) acide 4-[3-(1-adamantyl)-4-methoxyethoxymethoxybenzamido]benzoïque.

Dans un tricol et sous courant d'azote, on introduit 1,7 g (3,2 mmoles) de l'ester allylique précédent 50 ml de THF et 200 mg de tétrakis(triphényphosphine)Pd(0). On ajoute goutte à goutte une solution du sel de sodium du malonate de diéthyle, préparé à partir de 105 mg (3,5 mmoles) d'hydrure de sodium (80% dans l'huile) et 500 µl (3,2 mmoles) de malonate de diéthyle et agite à température ambiante pendant 2 heures. On évapore à sec le milieu réactionnel, reprend par l'eau, acidifie à pH 4, extrait avec de l'éther éthylique, décante la phase organique, sèche sur sulfate de magnésium, évapore. Le résidu obtenu est purifié par chromatographie sur colonne de silice élué avec de l'éther éthylique. On recueille 1,1 g (70%) de l'acide attendu de point de fusion 150-2°C.

### EXEMPLE 11

### Acide 4-[3-(1-adamantvl)-4-methoxyethoxymethoxybenzoyloxy]benzoïque.

### (a) 4-[3-(1-adamantyl)-4-methoxyethoxymethoxybenzoyloxy]benzoate d'allyle.

De manière analogue à l'exemple 10(d) par réaction de 1,5 g (3,9 mmoles) de chlorure de 3-(1-adamantyl)-4-méthoxyéthoxyméthoxybenzoyle avec 705 mg (3,9 mmoles) de 4-hydroxybenzoate d'allyle, on obtient 1,45 g (70%) de l'ester allylique attendu sous forme d'une huile jaune.

### (b) acide 4-[3-(1-adamantyl)-4-methoxyethoxymethoxybenzoyloxy]benzoïque.

De manière analogue à l'exemple 10(e) à partir de 1,45 g (2,8 mmoles) de l'ester allylique précédent, on obtient 870 mg (65%) de l'acide attendu de point de fusion 211-3°C.

### EXEMPLE 12

### Acide (E)-4-[[3-oxo-3-[3-(1-adamantyl)-4-methoxyethoxymethoxyphenyl]-1-propenyl]]benzoïque.

### (a) 3-(1-adamantyl)-4-méthoxyéthoxyméthoxyacétophenone.

Dans un tricol et sous courant d'azote on introduit 15,5 g (43 mmoles) d'acide 3-(1-adamantyl)-4-méthoxyéthoxyméthoxybenzoïque et 300 ml d'éther éthylique anhydre. A -20°C, on ajoute goutte à goutte 80 ml (0,13 mole)de méthyllithium (1,6 M dans l'éther) puis agite pendant trois heures à température ambiante. On verse le milieu réactionnel dans une solution aqueuse saturée en chlorure d'ammonium, décante la phase organique, sèche sur sulfate de magnésium, évapore. On recueille 15,4 g (100%) de l'acetophenone attendu sous forme d'une huile légèrement jaune.

### (b) (E)-4-[[3-oxo-3-[3-(1-adamantyl)-4-methoxyethoxymethoxyphenyl]-1-propenyl]]benzoate de méthyle.

Dans un ballon, on introduit 1,8 g (5 mmoles) de 3-(1-adamantyl)-4-méthoxyéthoxyméthoxyacétophenone 820 mg (5 mmoles) de 4-formylbenzoate de méthyle et 20 ml de méthanol. On ajoute 10 mg de 18-crown-6 et 200 mg de soude en pastille et agite à température ambiante pendant 4 heures. On évapore à sec et purifie le résidu obtenu par chromatographie sur colonne de silice élué avec du dichlorométhane. On recueille 1,4 g (55%) de l'ester méthylique attendu.

### (c) acide (E)-4-[[3-oxo-3-[3-(1-adamantyl)-4-methoxyethoxymethoxyphenyl]-1-propenyl]]benzoïque.

De manière analogue à l'exemple 2(a) à partir de 700 mg (1,4 mmole) de (E)-4-[[3-oxo-3-[3-(1-adamantyl)-4-methoxyethoxymethoxyphenyl]-1-propenyl]]benzoate de méthyle, on obtient 590 mg (87%) de l'acide attendu de point de fusion 154-6°C.

### EXEMPLE 13

### Acide -4-[[3-hydroxy-3-[3-(1-adamantyl)-4-methoxyethoxymethoxyphenyl]-1-propynyl]]benzoïque.

### (a) 3-(1-adamantyl)-4-methoxyethoxymethoxyphenylcarboxaldéhyde.

Dans un tricol et sous courant d'azote, on introduit 34 g (89 mmoles) de 3-(1-adamantyl)-1-bromo-4-méthoxyéthoxyméthoxybenzène et 250 ml de THF. A-78°C on ajoute goutte à goutte 43 ml (106 mmoles) d'une solution de n-butyllithium (2,5 M dans l'hexane) et agite 30'. On ajoute ensuite goutte à goutte 8,3 ml (106 mmoles) de DMF et laisse remonter à température ambiante. On verse le milieu réactionnel dans une solution aqueuse de chlorure d'ammonium, extrait avec de l'éther éthylique, décante la phase organique, sèche sur sulfate de magnésium, évapore. Le résidu obtenu est purifié par chromatographie sur colonne de silice élué avec du dichlorométhane. Après évaporation des solvants, on recueille 17,6 g (58%) d'aldéhyde attendu de point de fusion 63-4°C.

### (b) a-Triméthylsilyléthynyl-3-(1-adamantyl)-4-methoxyethoxymethoxyphenyl méthanol.

Dans un tricol, on introduit 1,7 ml (11,9 mmoles) de triméthylsilylacétylène et 25 ml de THF. A -78°C et sous courant d'azote, on ajoute goutte à goutte une solution de 5 ml (11,9 mmoles) de n-butyllithium (2,5 M dans l'hexane) et laisse revenir à température ambiante.
Cette solution est introduite goutte à goutte dans une solution de 3,7 g (10,7 mmoles) de 3-(1-adamantyl)-4-methoxyethoxymethoxyphenylcarboxaldéhyde dans 50 ml de THF à -78°C. On laisse le milieu réactionnel revenir à température ambiante, le verse dans une solution aqueuse de chlorure d'ammonium, extrait avec de l'éther éthylique, décante la phase organique, sèche sur sulfate de magnésium, évapore. On obtient 4,7 g (97%) de l'alcool attendu sous forme d'une huile jaune.

### (c) a-éthynyl-3-(1-adamantyl)-4-methoxyethoxymethoxyphenylméthanol.

Dans un ballon, on introduit 4,7 g (10,5 mmoles) d'a-Triméthylsilyléthynyl-3-(1-adamantyl)-4-methoxyethoxymethoxyphenylméthanol 50 ml de THF et ajoute goutte à goutte 11,4 ml (12,6 mmoles) d'une solution de fluorure de tétrabutylammonium (1,1 M dans le THF). On agite à température ambiante une heure, verse le milieu réactionnel dans l'eau, extrait avec de l'éther éthylique, décante la phase organique, sèche sur sulfate de magnésium, évapore.Le résidu obtenu est purifié par chromatographie sur colonne de silice élué avec un mélange d'acétate d'éthyle et d'heptane (30-70). Après évaporation des solvants, on recueille 2,5 g (64%) d'a-éthynyl-3-(1-adamantyl)-4-methoxyethoxymethoxyphenylméthanol.

### (d) 4-[[3-hydroxy-3-[3-(1-adamantyl)-4-methoxyethoxymethoxyphenyl]-1-propynyl]]benzoate de méthyle.

Dans un tricol, on introduit 2,5 g (6,7 mmoles) d'a-éthynyl-3-(1-adamantyl)-4-methoxyethoxymethoxyphenylméthanol, 1,2 g (6,7 mmoles) de 4-iodobenzoate de méthyle et 50 ml de triéthylamine. On dégaze le milieu réactionnel avec de l'azote pendant 30', puis ajoute successivement 380 mg (0,5 mmole) de Bis(triphénylphosphine)palladium(II)chlorure et 190 mg (0,8 mmole) d'iodure de cuivre. On agite à température ambiante pendant quatre heures, évapore à sec le milieu réactionnel, reprend le résidu obtenu par l'eau et l'éther éthylique. On décante la phase organique, sèche sur sulfate de magnésium, évapore. Le résidu obtenu est purifié par chromatographie sur colonne de silice élué avec un mélange d'acétate d'éthyle et d'heptane (20-80). On recueille 2,7 g (79%) de 4-[[3-hydroxy-3-[3-(1-adamantyl)-4-methoxyethoxymethoxyphenyl]-1-propynyl]]benzoate de méthyle de point de fusion 95-6°C.

### (e) acide 4-[[3-hydroxy-3-[3-(1-adamantyl)-4-methoxyethoxymethoxyphenyl]-1-propynyl]]benzoïque.

De manière analogue à l'exemple 9(c) à partir de 2,66 g (5,3 mmoles) de l'ester méthylique précédent, on obtient1,52 g (88%) de l'acide attendu de point de fusion 148-9°C.

### EXEMPLE 14

### Acide 4-[3-(1-adamantyl)-4-(2,2-diméthyl-1,3-dioxolanne-4-méthyloxy) phenylcarboxamido]benzoïque.

### (a) 3-(1-adamantyl)-4-(2,2-diméthyl-13-dioxolanne-4-méthyloxy)benzoate de méthyle.

A une solution de 10 g (0,0349 mole) de 3-(1-adamantyl)-4-hydroxybenzoate de méthyle dans 100 ml de DMF contenant 5,31 g (0,0384 moles) de carbonate de potassium, on ajoute goutte à goutte une solution de 12 g (0,0419 mole) de 3-tosyloxy-1,2-propanediolacetonide. Le milieu réactionnel est chauffé à 100°C durant douze heures puis versé dans de l'eau glacée et extrait avec de l'éther éthylique. On décante la phase organique, sèche sur sulfate de sodium, évapore. Le résidu est purifié par chromatographie sur colonne de silice élué avec un mélange d'hexane et d'acétate d'éthyle (80-20). Après évaporation des solvants, on recueille 8,8 g (63%) du produit attendu de point de fusion 158-9°C.

### (b) acide 3-(1-adamantyl)-4-(2,2-diméthyl-1,3-dioxolanne-4-méthyloxy)benzoïque.

Dans un ballon, on introduit 8,8 g (0,022 mole) de l'ester méthylique précédent et 200 ml d'une solution de soude méthanolique (2N). On chauffe à reflux pendant trois heures, et évapore à sec. On reprend le résidu par un mélange éther éthylique-eau, acidifie à pH 3, décante la phase organique, sèche sur sulfate de magnésium, évapore. On triture le solide obtenu dans l'hexane, filtre, sèche. On recueille 8,3 g (98%) d'acide attendu de point de fusion 224-5°C.

### (c) chlorure de 3-(1-adamantyl)-4-(2,2-diméthyl-1,3-dioxolanne-4-méthyloxy) benzoyle.

Dans un ballon; on introduit 8,3 g (0,0215 mole) d'acide 3-(1-adamantyl)-4-(2,2-diméthyl-1,3-dioxolanne-4-méthyloxy)benzoïque 80 ml de dichlorométhane et 4,3 ml (0,0216 mole) de dicyclohexylamine. On agite une heure à température ambiante, puis introduit 1,7 ml (0,0236 mole) de chlorure de thionyle. On agite pendant quatre heures, évapore à sec, reprend par l'éther éthylique, filtre le sel de dicyclohexylamine, évapore le filtrat et recueille 8,5 g (100%) de chlorure d'acide qui sera utilisé tel quel pour la suite de la synthese.

### (d) 4-[3-(1-adamantyl)-4-(2,2-diméthyl-1,3-dioxolanne-4-méthyloxy) phenylcarboxamido]benzoate de méthyle.

Dans un ballon, on introduit 1,62 g (10,7 mmoles) de 4-aminobenzoate de méthyle 30 ml de THF et 1,65 ml (11,8 mmoles) de triéthylamine; On ajoute goutte à goutte une solution de 4,3 g (10,7 mmoles) de chlorure de 3-(1-adamantyl)-4-(2,2-diméthyl-1,3-dioxolanne-4-méthyloxy) benzoyle dans 30 ml de THF et agite à température ambiante pendant seize heures. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec un mélange de dichlorométhane et d'éther éthylique (98-2). On recueille 3,1 g (56%) de l'ester méthylique attendu de point de fusion 174-5°C.

### (e) acide 4-[3-(1-adamantyl)-4-(2,2-diméthyl-1,3-dioxolanne-4-méthyloxy) phenylcarboxamido]benzoïque.

De manière analogue à l'exemple 14(b) à partir de 1 g (1,92 mmole) de l'ester méthylique précédent, on recueille 900 mg (93%) d'acide 4-[3-(1-adamantyl)-4-(2,2-diméthyl-1,3-dioxolanne-4-méthyloxy)phenylcarboxamido]benzoïque de point de fusion 245-6°C.

### EXEMPLE 15

### Acide 4-[3-(1-adamantyl)-4-(2,3-dihydroxypropyloxy)phenylcarboxamido] benzoïque.

### (a) 4-[3-(1-adamantyl)-4-(2,3-dihydroxypropyloxy)phenylcarboxamido] benzoate de méthyle.

Dans un ballon, on introduit 1,96 g (3,78 mmoles) de 4-[3-(1-adamantyl)-4-(2,2-diméthyl-1,3-dioxolanne-4-méthyloxy) phenylcarboxamido]benzoate de méthyle 70 ml de dichlorométhane 8 ml de THF et ajoute 7,24 g (38 mmoles) d'acide paratoluènesulfonique. On agite à température ambiante pendant quatre heures, verse le milieu réactionnel dans l'eau, extrait avec de l'éther éthylique, décante la phase organique, sèche sur sulfate de magnésium, évapore. On purifie le résidu obtenu par chromatographie sur colonne de silice élué avec un mélange d'acétate d'éthyle et de dichlorométhane (90-10). On recueille 1,46 g (81%) du produit attendu de point de fusion 111-2°C.

### (b) acide 4-[3-(1-adamantyl)-4-(2,3-dihydroxyprnpyloxy)phenylcarboxamido] benzoïque.

De manière analogue à l'exemple 14(b) à partir de 1,42 g (2,96 mmoles) de l'ester méthylique précédent, on recueille 650 mg (47%) d' acide 4-[3-(1-adamantyl)-4-(2,3-dihydroxypropyloxy)phenylcarboxamido]benzoïque de point de fusion 236-7°C.

### EXEMPLE 16

### Acide 4-[3-(1-adamantyl)-4-méthoxycarbonylméthyloxyphénylcarboxamido] benzoïque.

### (a) 3-(1-adamantyl)-4-méthoxycarbonylméthyloxybenzoate de benzyle.

Dans un tricol, on introduit 2,1 g (5,79 mmoles) de 3-(1-adamantyl)-4-hydroxy benzoate de benzyle et 20 ml de DMF et ajoute par petites quantités 191 mg (6,34 mmoles) d'hydrure de sodium (80% dans l'huile). On agite jusqu'à cessation du dégagement gazeux puis ajoute 565 µl (5,79 mmoles) de bromoacétate de méthyle et agite à température ambiante douze heures. On verse le milieu réactionnel dans l'eau, extrait avec de l'éther éthylique, décante la phase organique, sèche sur sulfate de magnésium, évapore. On triture le solide dans l'hexane, filtre, sèche. On recueille 2,36 g (94%) de produit attendu de point de fusion 134-5°C.

### (b) acide 3-(1-adamantyl)-4-méthoxycarbonylméthyloxybenzoïque.

Dans un réacteur, on introduit 2,34 g (5,38 mmoles) de 3-(1-adamantyl)-4-méthoxycarbonylméthyloxybenzoate de benzyle 40 ml de dioxanne 500 µl d'acide acétique et 234 mg de palladium (10%). On hydrogène à 40°C et sous une pression de 7 bars pendant trois heures. On filtre le catalyseur, évapore à sec. On triture le solide obtenu dans l'hexane, filtre, sèche. On recueille 1,74 g (94%) de l'acide attendu de point de fusion 230-1°C.

### (c) chlorure de 3-(1-adamantyl)-4-méthoxycarbonylméthyloxybenzoyle.

Dans un ballon, on introduit 1,78 g (5 mmoles) d'acide 3-(1-adamantyl)-4-méthoxycarbonylméthyloxybenzoïque 15 ml de chlorure de thionyle et chauffe à reflux pendant une heure. On évapore à sec et recueille le chlorure d'acide brut qui sera utilisé tel quel pour la suite de la synthèse.

### (d) 4-[3-(1-adamantyl)-4-méthoxycarbonylméthyloxyphénylcarboxamido]benzoate de benzyle.

De manière analogue à l'exemple 14(d) par réaction de 1,13 g (5 mmoles) de 4-aminobenzoate de benzyle avec 1,8 g (5 mmoles) de chlorure de 3-(1-adamantyl)-4-méthoxycarbonylméthyloxybenzoyle, on obtient 1,7 g (61%) de l'ester benzylique attendu de point de fusion 95-6°C.

### (e) acide 4-[3-(1-adamantyl)-4-méthoxycarbonylméthyloxyphénylcarboxamido] benzoïque.

De manière analogue à l'exemple 16(b) à partir de 1,69 g (3 mmoles) de 4-[3-(1-adamantyl)-4-méthoxycarbonylméthyloxyphénylcarboxamido]benzoate de benzyle, on obtient 1,13 g (80%) d'acide attendu de point de fusion 291-2°C.

### EXEMPLE 17

### Acide 2-[3-(1-adamantyl)-4-méthoxyphénoxyméthyl]-4-thiophenecarboxytique.

### (a) 2-[3-(1-adamantyl)-4-méthoxyphénoxyméthyl]-4-thiophenecarboxylate de méthyle.

Dans un tricol et sous courant d'azote, on introduit 210 mg (7 mmoles) d'hydrure de sodium (80% dans l'huile) et 10 ml de DMF. On ajoute goutte à goutte une solution de 1,8 g (7 mmoles) de 3-(1-adamantyl)-4-méthoxyphenol dans 20 ml de DMF et agite jusqu'à cessation du dégagement gazeux. On ajoute ensuite une solution de 1,7 g (7,2 mmoles) de 2-bromomethyl-4-thiophenecarboxylate de methyle dans 15 ml de DMF et agite à température ambiante pendant deux heures. On verse le milieu réactionnel dans l'eau, extrait avec de l'éther éthylique, décante la phase organique, sèche sur sulfate de magnésium, évapore. Le solide obtenu est trituré dans l'hexane, filtré, sèché. On recueille 2,6 g (90%) de l'ester méthylique attendu de point de fusion 97-8°C.

### (b) acide 2-[3-(1-adamantyl)-4-méthoxyphénoxyméthyl]-4-thiophenecarboxylique.

De manière analogue à l'exemple 14(b) à partir de 2 g (5,6 mmoles) de l'ester méthylique précédent, on obtient 1,7 g (88%) d'acide 2-[3-(1-adamantyl)-4-méthoxyphénoxyméthyl]-4-thiophenecarboxylique de point de fusion 198-200°C.

### EXEMPLE 18

### Acide 2-[3-(1-adamantyl)-4-méthoxyphénylaminométhyl]-4-thiophene carboxulique.

### (a) 2-[3-(1-adamantyl)-4-méthoxyphénylaminomethyl]-4-thiophenecarboxylate de méthyle.

De manière analogue à l'exemple 17(a) par réaction de 2,4 g (6,8 mmoles) de 3-(1-adamantyl)-4-méthoxytrifluoroacetanilide avec 1,6 g (6,8 mmoles) de 2-bromomethyl-4-thiophenecarboxylate de methyle, on obtient 2,6 g (75%) de l'ester méthylique attendu sous forme d'une huile jaune.

### (b) acide 2-[3-(1-adamantyl)-4-méthoxyphénylaminométhyl]-4-thiophene carboxylique.

De manière analogue à l'exemple 14(b) à partir de 2,6 g (5,1 mmoles) de l'ester méthylique précédent, on obtient 1,5 g (74%) d'acide 2-[3-(1-adamantyl)-4-méthoxyphénylaminométhyl]-4-thiophene carboxylique de point de fusion 212-4°C.

### EXEMPLE 19

### Acide 2-[3-(1-adamantyl)-4-méthoxyphénylthiométhyl]-4-thiophene carboxylique.

### (a) 2-[3-(1-adamantyl)-4-méthoxyphénylthiomethyl]-4-thiophenecarboxylate de méthyle.

De manière analogue à l'exemple 17(a) par réaction de 800 mg (2,9 mmoles) de 3-(1-adamantyl)4-méthoxythiophénol avec 690 mg (2,9 mmoles) de 2-bromomethyl-4-thiophenecarboxylate de methyle, on obtient 700 mg (56%) de l'ester méthylique attendu sous forme d'une huile incolore.

### (b) acide 2-[3-(1-adamantyl)-4-méthoxyphénylthiométhyl]-4-thiophene carboxylique.

De manière analogue à l'exemple 14(b) à partir de 700 mg (1,6 mmole) de l'ester méthylique précédent, on obtient 460 mg (68%) d' acide 2-[3-(1-adamantyl)-4-méthoxyphénylthiométhyl]-4-thiophene carboxylique de point de fusion 154-6°C.

### EXEMPLE 20

### Acide 4-[3-(1-adamantyl)-4-(2,2-diméthyl-1,3-dioxolanne-4-méthyloxy) benzoyloxy]benzoïque.

### (a) 4-[3-(1-adamantyl)-4-(2,2-diméthyl-1,3-dioxolanne-4-méthyloxy) benzoyloxy]benzoate d'allyle.

De manière analogue à l'exemple 14(d) par réaction de 4,3 g (10,7 mmoles) de chlorure de 3-(1-adamantyl)-4-(2,2-diméthyl-1,3-dioxolanne-4-méthyloxy) benzoyle avec 1,9 g (10,7 mmoles) de 4-hydroxybenzoate d'allyle, on obtient 4,24 g (72%) de l'ester allylique attendu de point de fusion 106-7°C.

### (b) acide 4-[3-(1-adamantyl)-4-(2,2-diméthyl-1,3-dioxolanne-4-méthyloxy) benzoyloxy]benzoïque.

Dans un tricol et sous courant d'azote, on introduit 1 g (1,83 mmole) de l'ester allylique précédent 10 ml de THF et 104 mg de tétrakis(triphénylphosphine) palladium (0). On ajoute goutte à goutte 1,6 ml (18,3 mmoles) de morpholine et agite à température ambiante pendant trois heures. On verse le milieu réactionnel dans l'eau glacée,acidifie à pH 3, extrait avec de l'éther éthylique, décante la phase organique, sèche sur sulfate de magnésium,évapore. Le résidu obtenu est purifié par chromatographie sur colonne de silice élué avec un mélange de dichlorométhane et d'acétate d'éthyle (50-50). Après évaporation des solvants, on recueille 435 mg (47%) d'acide 4-[3-(1-adamantyl)-4-(2,2-diméthyl-1,3-dioxolanne-4-méthyloxy) benzoyloxy]benzoïque de point de fusion 238-40°C.

### EXEMPLE 21

### Acide 4-[3-hydroxy-3-[5-(1-adamantyl)-2,4-dimethoxyethoxymethoxyphenyl]-1-pronynyl]benzoïque.

### (a) 5-(1-adamantyl)-2,4-dihydroxybenzaldehyde.

Dans un ballon, on introduit 40 g (0,29 mole) de 2,4-dihydroxybenzaldehyde 600 ml de dichlorométhane 46,4 g (0,34 mole) de 1-adamantanol et ajoute 24 ml d'acide sulfurique concentré. On agite à température ambiante pendant douze heures, verse le milieu réactionnel dans l'eau, décante la phase organique, sèche sur sulfate de magnésium, évapore. Le résidu obtenu est purifié par chromatographie sur colonne de silice élué avec du dichlorométhane. On recueille 50,8 g (64%) de l'aldéhyde attendu de point de fusion 259-61°C.

### (b) 5-(1-adamantyl)-2,4-di-methoxyethoxymethoxybenzaldehyde.

De manière analogue à l'exemple 17(a) par réaction de 25 g (91,9 mmoles) de 5-(1-adamantyl)-2,4-dihydroxybenzaldehyde avec 26,2 ml (0,23 mole) de chlorure de 2-méthoxyéthoxyméthyle, on obtient 31 g (75%) de produit attendu sous forme d'une huile jaune.

### (c) a-Triméthylsilyléthynyl-5-(1-adamantyl)-2,4-di-methoxyethoxymethoxy benzeneméthanol.

Dans un tricol, on introduit 450 µl (3,2 mmoles) de triméthylsilylacétylène et 50 ml de THF. A -78°C et sous courant d'azote, on ajoute goutte à goutte une solution de 1,3 ml (3,2 mmoles) de n-butyllithium (2,5 M dans l'hexane) et laisse revenir à température ambiante.
Cette solution est introduite goutte à goutte dans une solution de 1,3 g (2,9 mmoles) de 5-(1-adamantyl)-2,4-di-methoxyethoxymethoxybenzaldehyde dans 50 ml de THF à -78°C. On laisse le milieu réactionnel revenir à température ambiante, le verse dans une solution aqueuse de chlorure d'ammonium, extrait avec de l'éther éthylique, décante la phase organique, sèche sur sulfate de magnésium, évapore. On obtient 1,6 g (100%) de l'alcool attendu sous forme d'une huile jaune.

### (d) a-éthynyl-5-(1-adamantyl)-2,4-di-methoxyethoxymethoxybenzeneméthanol.

Dans un ballon, on introduit 1,6 g (2,9 mmoles) de d'a-Triméthylsilyléthynyl-5-(1-adamantyl)-2,4-di-methoxyethoxymethoxybenzeneméthanol 50 ml de THF et ajoute goutte à goutte 3,2 ml (3,5 mmoles) d'une solution de fluorure de tétrabutylammonium (1,1 M dans le THF). On agite à température ambiante une heure, verse le milieu réactionnel dans l'eau, extrait avec de l'éther éthylique, décante la phase organique, sèche sur sulfate de magnésium, évapore.Le résidu obtenu est purifié par chromatographie sur colonne de silice élué avec un mélange de dichlorométhane et d'éther éthylique (95-5). Après évaporation des solvants, on recueille 760 mg (55%)de produit attendu sous forme d'une huile jaune.

### (e) 4-[3-hydroxy-3-[5-(1-adamantyl)-2,4-di-methoxyethoxymethoxyphenyl]-1-propynyl]benzoate de méthyle.

Dans un tricol, on introduit 760 mg (1,6 mmole) d'a-éthynyl-5-(1-adamantyl)-2,4-di-methoxyethoxymethoxybenzeneméthanol, 420 mg (1,6 mmole) de 4-iodo benzoate de méthyle et 20 ml de triéthylamine. On dégaze le milieu réactionnel avec de l'azote pendant 30', puis ajoute successivement 90 mg de Bis(triphényl phosphine)palladium(II)chlorure et 37 mg d'iodure de cuivre. On agite à température ambiante pendant quatre heures, évapore à sec le milieu réactionnel, reprend le résidu obtenu par l'eau et l'éther éthylique. On décante la phase organique, sèche sur sulfate de magnésium, évapore. Le résidu obtenu est purifié par chromatographie sur colonne de silice élué avec du dichlorométhane, on recueille 800 mg (82%) de l'ester méthylique attendu sous forme d'une huile marron.

### (f) acide 4-[3-hydroxy-3-[5-(1-adamantyl)-2,4-di-methoxyethoxymethoxyphenyl]-1-propynyl]benzoïque.

Dans un ballon, on introduit 800 mg (1,3 mmole) de l'ester méthylique précédent 340 mg (7,8 mmoles) d'hydroxyde de lithium et 50 ml de THF et chauffe à reflux pendant douze heures. On évapore à sec, reprend par eau, acidifie à pH 1, extrait avec de l'éther éthylique, décante la phase organique, sèche sur sulfate de magnésium, évapore. Le résidu obtenu est purifié par chromatographie sur colonne de silice élué avec de l'éther éthylique. On recueille 420 mg (54%) d'acide 4-[3-hydroxy-3-[3-(1-adamantyl)-2,5-di-methoxyethoxymethoxyphenyl]-1-propynyl] benzoïque de point de fusion 79-81°C.

### EXEMPLE 22

### Acide 4-[3-(1-adamantyl)-4-methoxyethoxymethoxybenzoylthio]benzoïque.

Dans un ballon, on introduit 1,54 g (10 mmoles) d'acide 4-mercaptobenzoïque et 40 ml de pyridine. On ajoute goutte à goutte une solution de 3,75 g (10 mmoles) de chlorure de 3-(1-adamantyl)-4-méthoxyéthoxyméthoxybenzoyle préparé à l'exemple 10(c) et agite à température ambiante pendant six heures. On évapore à sec, reprend par eau et éther éthylique, décante la phase organique, lave à l'eau, sèche sur sulfate de magnésium, évapore. Le solide obtenu est recristallisé dans un mélange éther diisopropylique-méthanol, filtré, séché. On recueille 2,94 g (59%) d'acide 4-[3-(1-adamantyl)-4-methoxyethoxymethoxybenzoylthio]benzoïque de point de fusion 187-8°C.

### EXEMPLE 23

### Acide N-methyl-4-[3-(1-adamantyl)-4-methoxyethoxymethoxyphenyl carboxamido]benzoïque.

### (a) N-methyl-4-[3-(1-adamantyl)-4-methoxyethoxymethoxybenzamido]benzoate d'allyle

De manière analogue à l'exemple 16(a) par réaction de 1,35 g (2,6 mmoles) de 4-[3-(1-adamantyl)-4-methoxyethoxymethoxybenzamido]benzoate d'allyle préparé à l'exemple 10(d) avec 190 µl (3,1 mmole) d'iodomethane, on obtient 1,38 g (100%) de l'ester allylique attendu.

### (b) acide N-methyl-4-[3-(1-adamantyl)-4-methoxyethoxymethoxyphenyl carboxamido]benzoïque.

De manière analogue à l'exemple 20(b) à partir de 1,32 g (2,5 mmoles) de l'ester allylique précédent, on obtient 740 mg (60%) d'acide N-methyl-4-[3-(1-adamantyl)-4-methoxyethoxymethoxyphenyl carboxamido]benzoïque de point de fusion 159-60°C.

### EXEMPLE 24

### Acide-4-[3-(1-adamantyl)-4-methoxyethoxymethoxyphenylthio carboxamido]benzoïque.

### (a) 4-[3-(1-adamantyl)-4-methoxyethoxymethoxyphenylthiocarboxamido]benzoate d'allyle.

De manière analogue à l'exemple 6(a) par réaction de 2 g (3,9 mmoles) de 4-[3-(1-adamantyl)-4-methoxyethoxymethoxybenzamido]benzoate d'allyle avec 940 mg (2,3 mmoles) de réactif de Lawesson, on obtient 1,93 g (92%) de l'ester allylique attendu.

### (b) acide -4-[3-(1-adamantyl)-4-methoxyethoxymethoxyphenylthiocarboxamido] benzoïque.

De manière analogue à l'exemple 20 (b) à partir de 1,9 g (3,5 mmoles) de l'ester allylique précédent, on obtient 50 mg (29%) d'acide -4-[3-(1-adamantyl)-4-methoxyethoxymethoxyphenylthiocarboxamido] benzoïque de point de fusion 98-9°C.

### EXEMPLE 25

### Acide (E)-4-[[3-oxo-3-[3-(1-adamantyl)-4-methoxymethoxyphenyl]-1-propenyl]]benzoïque.

### (a) 3-(1-adamantyl)-4-méthoxyméthoxybenzoate de méthyle.

Dans un tricol et sous courant d'azote, on introduit 9 g (0,3 mole) d'hydrure de sodium (80% dans l'huile) et 50 ml de DMF. On ajoute goutte à goutte une solution de 71,6 g (0,25 mole) de 3-(1-adamantyl)-4-hydroxybenzoate de méthyle dans 500 ml de DMF et agite jusqu'à cessation du dégagement gazeux. On ajoute ensuite 22,8 ml (0,3 mole) de chlorure de méthoxyméthyle et agite à température ambiante une heure. On verse le milieu réactionnel dans l'eau, extrait avec de l'éther éthylique, décante la phase organique, sèche sur sulfate de magnésium, évapore. On recueille 67,7 g (82%) du produit attendu sous forme d'une huile.

### (b) acide 3-(1-adamantyl)-4-méthoxyméthoxybenzoïque.

De maniére analogue à l'exemple 14(b) à partir de 67,7 g (0,2 mole) de l'ester méthylique précédent, on obtient 59 g (91%) d'acide 3-(1-adamantyl)-4-méthoxy méthoxybenzoïque.

### (c) 3-(1-adamantyl)-4-méthoxyméthoxyacétophenone.

Dans un tricol et sous courant d'azote on introduit 58 g (0,183 mole) d'acide 3-(1-adamantyl)-4-méthoxyméthoxybenzoïque et 500 ml d'éther éthylique anhydre. A -20°C, on ajoute goutte à goutte 252 ml (0,4 mole) de méthyllithium (1,6 M dans l'éther) puis agite pendant trois heures à température ambiante. On verse le milieu réactionnel dans une solution aqueuse saturée en chlorure d'ammonium, décante la phase organique, sèche sur sulfate de magnésium, évapore. On recueille 59,6 g (100%) de l'acetophenone attendu sous forme d'une huile légèrement jaune.

### (d) (E)-4-[[3-oxo-3-[3-(1-adamantyl)-4-methoxymethoxyphenyl]-1-propenyl]]benzoate de méthyle.

Dans un ballon, on introduit 40,9 g 0,13 mole) de 3-(1-adamantyl)-4-méthoxyméthoxyacétophenone 23,47 g (0,143 mole) de 4-formylbenzoate de méthyle et 600 ml de méthanol. On ajoute 24,8 ml (0,13 mole) d'une solution de methylate de sodium (5,25M) et agite à température ambiante pendant 18 heures. On évapore à sec reprend par un mélange eau-acétate d'éthyle, décante la phase organique sèsche sur sulfate de magnésium, évapore. Le résidu obtenu est purifie par trituration dans un mélange d'hexane et d'acétate d'éthyle, filtré, séché. On recueille 46,1 g (77%) de (E)-4-[[3-oxo-3-[3-(1-adamantyl)-4-methoxymethoxy phenyl]-1-propenyl]]benzoate de méthyle de point de fusion 170-1°C.

### (e) acide (E)-4-[[3-oxo-3-[3-(1-adamantyl)-4-methoxymethoxyphenyl]-1-propenyl]]benzoïque.

De manière analogue à l'exemple 2(a) à partir de 5 g (10,8 mmoles) de (E)-4-[[3-oxo-3-[3-(1-adamantyl)-4-methoxymethoxyphenyl]-1-propenyl]]benzoate de méthyle, on obtient 3 g (62%) de l'acide attendu de point de fusion 205-6°C.

### EXEMPLE 26

### Acide (E)-4-[[3-oxo-3-[3-(1-adamantyl)-4-hydroxyphenyl]-1-propenyl]] benzoïque.

### (a) (E)-4-[[3-oxo-3-[3-(1-adamantyl)-4-hydroxyphenyl]-1-propenyl]]benzoate de méthyle.

Dans un ballon, on introduit 40,2 g (87,4 mmoles) de (E)4-[[3-oxo-3-[3-(1-adamantyl)-4-methoxymethoxyphenyl]-1-propenyl]]benzoate de méthyle un litre de méthanol et 300 ml de THF. On ajoute 50 ml d'acide sulfurique concentré et chauffe à reflux pendant quatre heures. On évapore le milieu réactionnel, reprend par un mélange acétate d'éthyle-eau, décante la phase organique, sèche sur sulfate de magnésium, évapore. Le solide obtenu est trituré dans un mélange acétate d'éthyle-hexane (30-70), filtré, séché. On recueille 32,1g (88%) de l'ester méthylique attendu de point de fusion 256-7°C.

### (b) acide (E)4-[[3-oxo-3-[3-(1-adamantyl)-4-hydroxyphenyl]-1-propenyl]]benzoïque.

De manière analogue à l'exemple 2(a) à partir de 5 g (12 mmoles) de l'ester méthylique précédent, on obtient 4,8 g (100%) d'acide (E)-4-[[3-oxo-3-[3-(1-adamantyl)-4-hydroxyphenyl]-1-propenyl]]benzoïque de point de fusion > 300°C avec décomposition.

### EXEMPLE 27

Dans cet exemple, on a illustré diverses formulations concrètes à base des composés selon l'invention.

### A- VOIE ORALE

(a) Comprimé de 0,2 g
   - Composé de l'exemple 1 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé de l'exemple 2 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé de l'exemple 6 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé de l'exemple 4 0,05 g
   - Glycérine 1,000g
   - Sorbitol à 70% 1,000g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé de l'exemple 1 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 6 0,300 g
   - Vaseline blanche codex 100 g
(c) Crème Eau-dans-Huile non ionique
   - Composé de l'exemple 1 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 1 0,100 g
   - Polyéthylène glycol (PEG 400) 69,900 g
   - Ethanol à 95% 30,000 g
(e) Onguent hydrophobe
   - Composé de l'exemple 2 0,300 g
   - Mirystate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 cst" vendu par GOLDSCHMIDT) 100g
(f) Crème Huile-dans-Eau non ionique
   - Composé de l'exemple 4 0,500 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile 100 g

## Revendications

1. Composés bicycliques aromatiques, caractérisés par le fait qu'ils répondent à la formule générale (I) suivante : 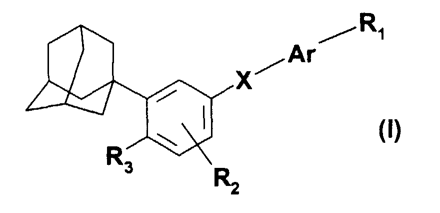 dans laquelle:
- R₁ représente
(i) le radical -CH₃
(ii) le radical -(CH₂)n-O-R₄
(iii) le radical -O-(CH₂)m-(CO)n-R₅
(iv) le radical -CO-R₆
(v) le radical -CO-O-R₇
les valeurs m,n ainsi que les différents radicaux R₄ à R₇ ayant la signification donnée ci-après,
- R₂ représente un atome d'hydrogène, d'halogène, un radical alkyle linéaire ou ramifié ayant de 1 à 20 atomes de carbone, un radical -OR₄, un radical -O-CH₂-O-CH₂-CH₂-O-CH₃.
- R₃ représente:
(i) un radical -Y-(CH₂)p-Y-(CH₂)q-R₈
(ii) un radical -(CH₂)p-Y-(CH₂)q-R₈
(iii) un radical -Y-(CH₂)q-R₈
(iv) un radical -CH=CH-(CH₂)q-R₈
(v) un radical -(CH₂)q-R₈
les valeurs p, q, r et les radicaux Y et R₈ ayant la signification donnée ci-après.
- X représente les liaisons de formules (a)-(k) suivantes pouvant être lues dans les deux sens : 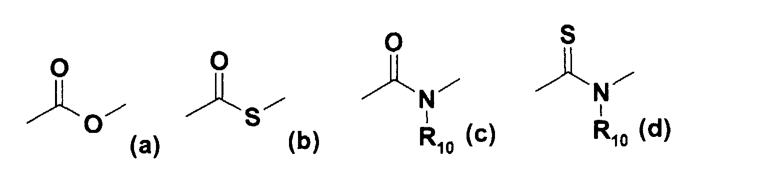 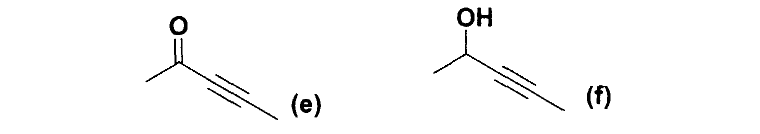 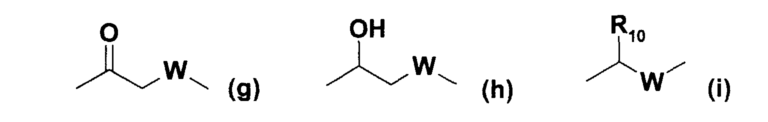 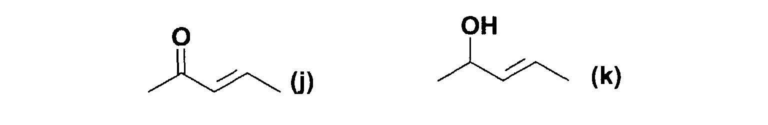
- Ar représente un radical choisi parmi les radicaux de formule (a)-(f) suivantes : 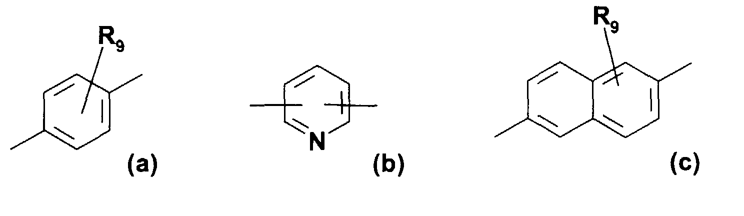 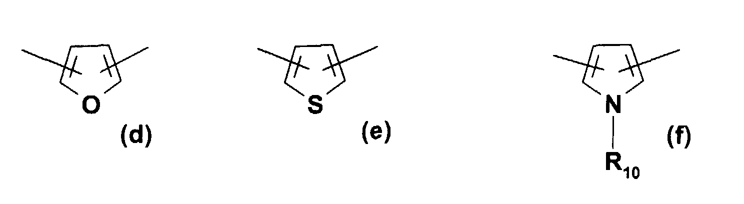 étant entendu que dans tout ce qui précède:
- m est un nombre entier égal à 1,2 ou 3
- n est un nombre entier égal à 0 ou 1
- p est un nombre entier compris inclusivement entre 1 et 12
- q est un nombre entier compris inclusivement entre 0 et 12
- r est un nombre entier compris inclusivement entre 0 et 10
- t est un nombre entier égal à 0, 1 ou 2
- Y représente l'atome d'oxygène ou le radical S(O)t
- W représente l'atome d'oxygène, le radical S(O)t ou le radical N-R₁₀
- R₄ représente un atome d'hydrogène, un radical alkyle inférieur ou un radical -CO-R₁₁
- R₅ représente un alkyle inférieur ou un hétérocycle
- R₆ représente un atome d'hydrogène, un radical alkyle inférieur ou un radical 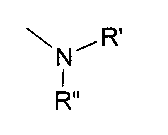 dans lequel R' et R" représentent un atome d'hydrogène, un radical alkyle inférieur, un radical mono ou polyhydroxyalkyle, un radical aryle éventuellement substitué ou un reste d'aminoacide ou de peptide ou de sucre ou encore pris ensemble, forment un hétérocycle.
- R₇ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide.
- R8, identique ou différent, représente un atome d'hydrogène, un radical alkyle ramifié ayant de 1 à 20 atomes de carbone, un radical cycloaliphatique en C3-C6, un radical monohydroxyalkyle ou un radical polyhydroxyalkyle dont les hydroxy sont éventuellement protégés sous forme de méthoxy ou d'acétoxy ou d'acétonide, un radical aryle, un radical alkynyle, un radical -CO-R₆, un radical -CO-O-R₇, un radical aminoalkyle dont la fonction amine est éventuellement substituée par un ou deux groupements alkyles inférieurs, un hétérocycle, R₇ ayant la signification donnée ci-dessus
- R₉, identique ou différent, représente un atome d'hydrogène, d'halogène, un radical allkyle linéaire ou ramifié ayant de 1 à 20 atomes de carbone, un radical -OR₄, un radical -O-CH₂-O-CH₂-CH₂-O-CH₃.
- R₁₀, identique ou différent, représente un atome d'hydrogène, un radical alkyle inférieur
- R₁₁ représente un radical alkyle inférieur
étant donné que lorsque:
- X représente la liaison de formule (a), (b), (c), (g), (h), (j) ou (k), alors R₃ est différent du radical de formule (iii), (iv) ou (v) dans laquelle R₈ est un atome d'hydrogène,
- X représente la liaison de formule (a), alors R₃ est différent du radical (iii),
- X représente la liaison de formule (b) et R₃ représente le radical de formule (iii) dans laquelle Y représente un atome de soufre, alors R₈ est différent du radical aryle,
- X représente la liaison de formule (g), (h), (j) ou (k) et R₃ représente le radical de formule (v), alors R₈ est différent d'un radical monohydroxyalkyle ou polyhydroxyalkyle, ou d'un radical cycloaliphatique en C3-C6.
ainsi que leurs sels et leurs isomères optiques et géométriques.

2. Composés selon la revendication 1, caractérisés par le fait qu'ils se présentents sous forme de sels d'un métal alcalin ou alcalino-terreux, ou encore de zinc ou d'une amine organique.

3. Composés selon l'une des revendications 1 ou 2, caractérisés par le fait que les radicaux alkyles inférieurs sont choisis parmi le groupe constitué par les radicaux méthyle, éthyle, isopropyle, butyle, tertiobutyle, hexyle, nonyle et dodécyle.

4. Composés selon l'une des revendications précédentes, caractérisés en ce que les radicaux alkyles linéaires ou ramifiés ayant de 1 à 20 atomes de carbone sont choisis parmi le groupe constitué par les radicaux méthyle, éthyle, propyle, 2-éthyl-hexyle, octyle, dodécyle, hexadécyle et octadécyle.

5. Composés selon l'une des revendications précédentes, caractérisés en ce que les radicaux monohydroxyalkyles sont choisis parmi le groupe constitué par les radicaux 2-hydroxyéthyle, 2-hydroxypropyle ou 3-hydroxypropyle.

6. Composés selon l'une des revendications précédentes, caractérisés en ce que les radicaux polyhydroxyalkyles sont choisis parmi le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une des revendications précédentes, caractérisés en ce que le radical aryle est un radical phényle éventuellement substitué par au moins un atome d'halogène, un hydroxyle ou une fonction nitro.

8. Composés selon l'une des revendications précédentes, caractérisés en ce que les radicaux aralkyles sont choisis parmi le groupe constitué par les radicaux benzyle ou phénéthyle, éventuellement substitués par au moins un atome d'halogène, un hydroxyle ou une fonction nitro.

9. Composés selon l'une des revendications précédentes, caractérisés en ce que les radicaux alkényles sont choisis dans le groupe constitué par les radicaux contenant de 1 à 5 atomes de carbone et présentant une ou plusieurs insaturations éthyléniques, et en particulier le radical allyle.

10. Composés selon l'une des revendications précédentes, caractérisés en ce que les restes de sucre sont choisis dans le groupe constitué par les restes de glucose, de galactose, de mannose et d'acide glucuronique.

11. Composés selon l'une quelconque des revendications précédentes, caractérisés en ce que les restes d'aminoacide sont choisis dans le groupe constitué par les restes dérivant de la lysine, de la glycine ou de l'acide aspartique.

12. Composés selon l'une quelconque des revendications précédentes, caractérisés en ce que les restes de peptides sont choisis dans le groupe constitué par les restes de dipeptides ou de tripeptides.

13. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Composés selon l'une quelconque des revendications précédentes, caractérisés en ce que les atomes d'halogène sont choisis dans le groupe constitué par le fluor, le chlore et le brome.

15. Composés selon l'une quelconque des revendications précédentes, caractérisés en ce que le radical alkyle ramifié ayant de 1 à 20 atomes de carbone est choisi parmi les radicaux 2-méthylbutyle, 2-méthylpentyle, 1-méthylhexyle, 3-méthylheptyle.

16. Composés selon l'une quelconque des revendications précédentes, caractérisés en ce que le radical aminoalkyle est choisi parmi les radicaux aminométhyle, 3-aminopropyle, 6-aminohexyle.

17. Composés selon l'une quelconque des revendications précédentes, caractérisés en ce que le radical alkynyle présente de 2 à 6 atomes de carbone.

18. Composés selon l'une quelconque des revendications précédentes, caractérisés en ce que les radicaux cycloaliphatiques de 3 à 6 atomes de carbone sont choisis parmi le radical cyclopropyle et le radical cyclohexyle.

19. Composés selon la revendication 1, caractérisés par le fait qu'ils sont pris, seuls ou en mélanges, dans le groupe constitué par :
Acide 4-[3-(1-adamantyl)-4-methoxyphénylthiométhyl]benzoïque.
Acide 4-[3-(1-adamantyl)-4-methoxyphénylsulfinylméthyl]benzoïque.
Acide 4-[3-(1-adamantyl)-4-methoxyphénylméthylsulfonyl]benzoïque.
Acide 4-[3-(1-adamantyl)-4-methoxyphénylméthylsulfinyl]benzoïque.
Acide 4-[3-(1-adamantyl)-4-methoxyphénylsulfonylméthyl]benzoïque.
Acide 4-[3-(1-adamantyl)-4-methoxyphénylthiocarboxamido]benzoïque.
Acide 4-[3-(1-adamantyl)-4-methoxyphénylméthylthio]benzoïque.
Acide 4-[3-(1-adamantyl)-4-methoxyphénylméthylamino]benzoïque.
Acide 4-[[3-oxo-3-[3-(1-adamantyl)-4-methoxyphényl]-1-propynyl]]benzoïque.
Acide 4-[3-(1-adamantyl)-4-methoxyethoxymethoxybenzamido]benzoïque.
Acide 4-[3-(1-adamantyl)-4-methoxyethoxymethoxybenzoyloxy]benzoïque.
Acide (E)-4-[[3-oxo-3-[3-(1-adamantyl)-4-methoxyethoxymethoxyphenyl]-1-propenyl]]benzoïque.
Acide 4-[[3-hydroxy-3-[3-(1-adamantyl)-4-methoxyethoxymethoxyphenyl]-1-propynyl]]benzoïque.
Acide 4-[3-(1-adamantyl)-4-(2,2-diméthyl-1,3-dioxolanne-4-méthyloxy) phenylcarboxamido]benzoïque.
Acide 4-[3-(1-adamantyl)-4-(2,3-dihydroxypropyloxy)phenylcarboxamido] benzoïque.
Acide 4-[3-(1-adamantyl)4-méthoxycarbonylméthyloxyphénylcarboxamido] benzoïque..
Acide 2-[3-(1-adamantyl)-4-méthoxyphénoxyméthyl]-4-thiophenecarboxylique.
Acide 2-[3-(1-adamantyl)-4-méthoxyphénylaminométhyl]-4-thiophene carboxylique.
Acide 2-[3-(1-adamantyl)-4-méthoxyphénylthiométhyl]-4-thiophene carboxylique.
Acide 4-[3-(1-adamantyl)-4-(2,2-diméthyl-1,3-dioxolanne-4-méthyloxy) benzoyloxy]benzoïque.
Acide 4-[3-hydroxy-3-[5-(1-adamantyl)-2,4-dimethoxyethoxymethoxyphenyl]-1-propynyl]benzoïque.
Acide 4-[3-(1-adamantyl)-4-methoxyethoxymethoxybenzoylthio]benzoïque.
Acide N-methyl-4-[3-(1-adamantyl)-4-methoxyethoxymethoxyphenyl carboxamido]benzoïque.
Acide -4-[3-(-adamantyl)-4-methoxyethoxymethoxyphenylthiocarboxamido] benzoïque.
Acide (E)-4-[[3-oxo-3-[3-(1-adamantyl)-4-methoxymethoxyphenyl]-1-propenyl]]benzoïque.
Acide (E)-4-[[3-oxo-3-[3-(1-adamantyl)-4-hyd roxyphenyl]-1-propenyl]] benzoïque.
Acide (E)-4-[[3-oxo-3-[3-(1-adamantyl)-4-methoxyphenyl]-1-propenyl]] benzoïque.
Acide (E)-4-[[3-oxo-3-[3-(1-adamantyl)-4-(3-hydroxypropyloxy)phenyl]-1-propenyl]]benzoïque.
4-[3-(1-adamantyl)-4-methoxyethoxymethoxybenzoyloxy]benzaldéhyde.
4-[3-(1-adamantyl)-4-methoxyethoxymethoxybenzoyloxy]benzeneméthanol.
(E)-N-éthyl-4-[[3-oxo-3-[3-(1-adamantyl)-4-methoxyethoxymethoxyphenyl]-1-propenyl]]benzamide.
(E)-N-(4-hydroxyphenyl)4-[[3-oxo-3-[3-(1-adamantyl)-4-methoxyethoxy methoxyphenyl]-1-propenyl]]benzamide.
(E)-4-[[3-oxo-3-[3-(1-adamantyl)-4-methoxyethoxymethoxyphenyl]-1-propenyl]]phenol.

20. Composés selon la revendication 1, caractérisés par le fait qu'ils présentent l'une au moins des caractéristiques suivantes :
- R₁ est le radical
-COO-R₇ ou -CO-R₆.
- R₃ est le radical
-Y-(CH₂)p-Y-(CH₂)q-R₈.
-CH₂)p-(Y)n-(CH₂)q-R₈.
-Y-(CH₂)q-R₈.
- X représente une liaison de formule (a), (e), (f), (j) ou (k).
- Ar représente le radical de formule (a) ou (b).

21. Composés selon l'une quelconque des revendications précédentes pour une utilisation comme médicament.

22. Composés selon la revendication 21 pour une utilisation comme médicament destiné au traitement des affections dermatologiques, rhumatismales, respiratoires, cardiovasculaires et ophtalmologiques.

23. Utilisation de l'un au moins des composés définis aux revendications 1 à 20 pour la fabrication d'un médicament destiné au traitement des affections dermatologiques, rhumatismales, respiratoires, cardiovasculaires et ophtalmologiques.

24. Composition pharmaceutique, caractérisée par le fait qu'elle comprend, dans un support pharmaceutiquement acceptable, au moins l'un des composés tels que définis à l'une quelconque des revendications 1 à 20.

25. Composition selon la revendication 24, caractérisée en ce que la concentration en composé(s) selon l'une des revendication 1 à 16 est comprise entre 0,001 % et 5 % en poids par rapport à l'ensemble de la composition.

26. Composition cosmétique, caractérisée par le fait qu'elle comprend, dans un support cosmétiquement acceptable, au moins l'un des composés tels que définis à l'une quelconque des revendications 1 à 20.

27. Composition selon la revendication 26, caractérisée en ce que la concentration en composé(s) selon l'une des revendications 1 à 20 est comprise entre 0,001 % et 3 % en poids par rapport à l'ensemble de la composition.

28. Utilisation d'une composition cosmétique telle que définie à l'une des revendications 26 ou 27 pour l'hygiène corporelle ou capillaire.

## Patentansprüche

1. Aromatische bicyclische Verbindungen,
dadurch gekennzeichnet, daß
sie der folgenden allgemeinen Formel (I) entsprechen: 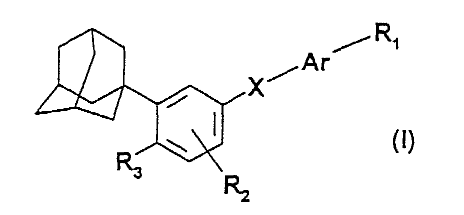 worin bedeuten:
- R₁:
(i) die Gruppe -CH₃
(ii) die Gruppe -(CH₂)ₙ-O-R₄
(iii) die Gruppe -O-(CH₂)m-(CO)n-R₅
(iv) die Gruppe -CO-R₆
(v) die Gruppe -CO-O-R₇,
wobei die Werte m und n sowie die verschiedenen Gruppe R₄ bis R₇ die nachstehend angegebenen Bedeutungen aufweisen,
- R₂: ein Wasserstoffatom, ein Halogenatom, eine geradkettige oder verzweigte Alkylgruppe mit 1 bis 20 Kohlenstoffatomen, eine Gruppe -OR₄, eine Gruppe -O-CH₂-O-CH₂-CH₂-O-CH₃,
- R₃:
(i) eine Gruppe -Y-(CH₂)p-Y-(CH₂)q-R₈
(ii) eine Gruppe -(CH₂)p-Y-(CH₂)q-R₈
(iii) eine Gruppe -Y-(CH₂)q-R₈
(iv) eine Gruppe -CH=CH-(CH₂)r-R₈
(v) eine Gruppe -(CH₂)q-R₈,
wobei die Werte p, q, r und die Gruppe Y und R₈ die nachfolgend angegebenen Bedeutungen aufweisen,
- X die Bindungen der folgenden Formeln (a) - (k), die in beiden Richtungen gelesen werden können: 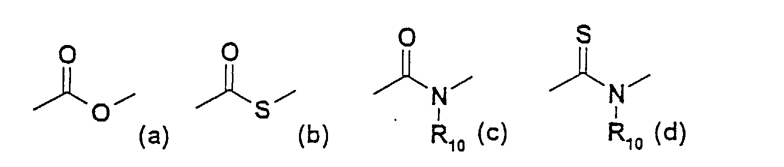 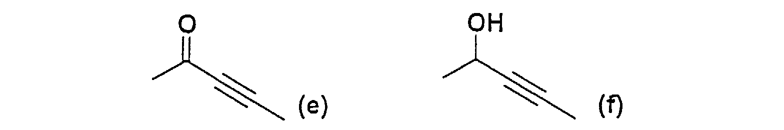 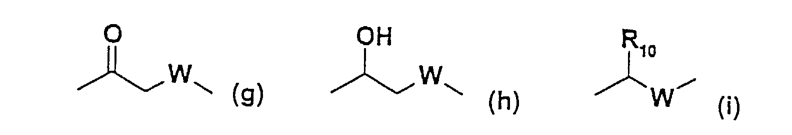 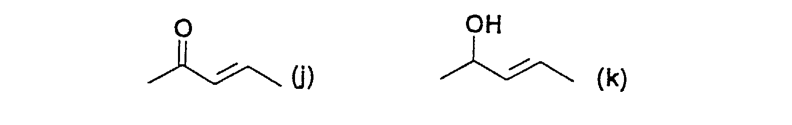
- Ar eine Gruppe, die unter den folgenden Gruppen der Formeln (a) - (f) ausgewählt ist: 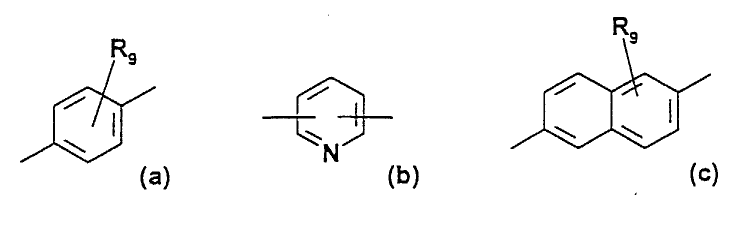 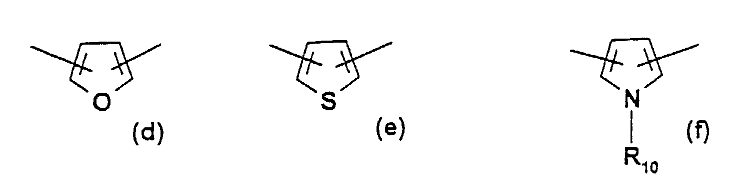
mit der Maßgabe, daß:
- m eine ganze Zahl 1, 2 oder 3 ist,
- n 0 oder 1 bedeutet,
- p eine ganze Zahl im Bereich von 1 bis 12 ist,
- q 0 oder eine ganze Zahl im Bereich von 1 bis 12 bedeutet,
- r 0 oder eine ganze Zahl im Bereich von 1 bis 10 bedeutet,
- t 0, 1 oder 2 ist,
- Y Sauerstoff oder die Gruppe S(O)t bedeutet,
- W Sauerstoff, die Gruppe S(O)t oder die Gruppe N-R₁₀ bedeutet,
- R₄ Wasserstoff, eine niedere Alkylgruppe oder eine Gruppe -CO-R₁₁ bedeutet,
- R₅ eine niedere Alkylgruppe oder einen Heterocyclus bedeutet,
- R₆ Wasserstoff, eine niedere Alkylgruppe oder eine Gruppe 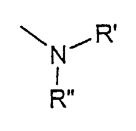 bedeutet, wobei R' und R" ein Wasserstoffatom, eine niedere Alkylgruppe, eine Mono- oder Polyhydroxyalkylgruppe, eine Arylgruppe, die gegebenenfalls substituiert ist, oder einen Aminosäurerest, Peptidrest oder Zuckerrest bedeuten oder gemeinsam einen Heterocyclus bilden,
- R₇ Wasserstoff, eine geradkettige oder verzweigte Alkylgruppe mit 1 bis 20 Kohlenstoffatomen, eine Alkenylgruppe, eine Mono- oder Polyhydroxyalkylgruppe, eine gegebenenfalls substituierte Aryl- oder Aralkylgruppe oder einen Zucker-, Aminosäure- oder Peptidrest bedeutet,
- die Gruppen R₈, die identisch oder verschieden sind, ein Wasserstoffatom, eine verzweigte Alkylgruppe mit 1 bis 20 Kohlenstoffatomen, eine cycloaliphatische Gruppe mit 3 bis 6 Kohlenstoffatomen, eine Monohydroxyalkylgruppe oder eine Polyhydroxyalkylgruppe, deren Hydroxygruppen gegebenenfalls in Form von Methoxy oder Acetoxy oder Acetonid geschützt sind, eine Arylgruppe, eine Alkinylgruppe, eine Gruppe -CO-R₆, eine Gruppe -CO-O-R₇, eine Aminoalkylgruppe, deren Aminogruppe gegebenenfalls mit einer oder zwei niederen Alkylgruppen substituiert ist, oder einen Heterocyclus bedeuten, wobei R₇ die oben angegebene Bedeutung aufweist,
- die Gruppen R₉, die identisch oder verschieden sind, ein Wasserstoffatom, ein Halogenatom, eine geradkettige oder verzweigte Alkylgruppe mit 1 bis 20 Kohlenstoffatomen, eine Gruppe -OR₄, eine Gruppe-O-CH₂-O-CH₂-CH₂-O-CH₃ bedeutet,
- die Gruppen R₁₀, die identisch oder voneinander verschieden sind, Wasserstoff oder eine niedere Alkylgruppe bedeuten,
- R₁₁ eine niedere Alkylgruppe bedeutet,
mit der Maßgabe, daß:
- R₃ von der Gruppe der Formel (iii), (iv) oder (v), worin R₈ Wasserstoff bedeutet, verschieden ist, wenn X die Bindung der Formel (a), (b), (c), (g), (h), (j) oder (k) bedeutet,
- R₃ von der Gruppe (iii) verschieden ist, wenn X die Bindung der Formel (a) bedeutet,
- R₈ von einer Arylgruppe verschieden ist, wenn X die Bindung der Formel (b) bedeutet und R3 die Gruppe der Formel (iii), worin Y ein Schwefelatom ist, bedeutet,
- R₈ von einer Monohydroxyalkylgruppe, einer Polyhydroxyalkylgruppe, oder einer cycloaliphatischen Gruppe mit 3 bis 6 Kohlenstoffatomen verschieden ist, wenn X die Bindung der Formel (g), (h), (j) oder (k) bedeutet und R₃ die Gruppe der Formel (v) ist;
und die Salze und die optischen und geometrischen Isomere dieser Verbindungen.

2. Verbindungen nach Anspruch 1, dadurch gekennzeichnet, daß sie in Form eines Alkali- oder Erdalkalimetallsalzes oder auch in Form eines Zinksalzes oder Salzes eines organischen Amins vorliegen.

3. Verbindungen nach einem der Ansprüche 1 oder 2, dadurch gekennzeichnet, daß die niederen Alkylgruppen unter Methyl, Ethyl, Isopropyl, Butyl, tert.-Butyl, Hexyl, Nonyl und Dodecyl ausgewählt sind.

4. Verbindungen nach einem der vorhergehenden Ansprüche, dadurch gekennzeichnet, daß die geradkettigen oder verzweigten Alkylgruppen mit 1 bis 20 Kohlenstoffatomen unter den Gruppen Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter den Gruppen 2-Hydroxyethyl, 2-Hydroxypropyl oder 3-Hydroxypropyl ausgewählt sind.

6. Verbindungen nach einem der vorhergehenden Ansprüche, dadurch gekennzeichnet, daß die Polyhydroxyalkylgruppen unter den Gruppen 2,3-Dihydroxypropyl, 2,3,4-Trihydroxybutyl, 2,3,4,5-Tetrahydroxypentyl oder Pentaerythrit ausgewählt sind.

7. Verbindungen nach einem der vorhergehenden Ansprüche, dadurch gekennzeichnet, daß die Arylgruppe eine Phenylgruppe ist, die gegebenenfalls mit mindestens einem Halogenatom, einer Hydroxygruppe oder einer Nitrogruppe substituiert ist.

8. Verbindungen nach einem der vorhergehenden Ansprüche, dadurch gekennzeichnet, daß die Aralkylgruppen unter den Gruppen Benzyl oder Phenethyl ausgewählt sind, die gegebenenfalls mit mindestens einem Halogenatom, einer Hydroxygruppe oder einer Nitrogruppe substituiert sind.

9. Verbindungen nach einem der vorhergehenden Ansprüche, dadurch gekennzeichnet, daß die Alkenylgruppen unter den Gruppen mit 1 bis 5 Kohlenstoffatomen, die eine oder mehrere ethylenisch ungesättigte Bindungen aufweisen, und insbesondere der Allylgruppe ausgewählt sind.

10. Verbindungen nach einem der vorhergehenden Ansprüche, dadurch gekennzeichnet, daß die Zuckerreste unter den Resten von Glucose, Galactose, Mannose und Glucuronsäure ausgewählt sind.

11. Verbindungen nach einem der vorhergehenden Ansprüche, dadurch gekennzeichnet, daß die Aminosäurereste unter den Resten ausgewählt sind, die von Lysin, Glycin oder Asparaginsäure abgeleitet sind.

12. Verbindungen nach einem der vorhergehenden Ansprüche, dadurch gekennzeichnet, daß die Peptidreste unter den Resten von Dipeptiden oder Tripeptiden ausgewählt sind.

13. Verbindungen nach einem der vorhergehenden Ansprüche, dadurch gekennzeichnet, daß die heterocyclischen Gruppen unter den Gruppen Piperidino, Morpholino, Pyrrolidino oder Piperazino, die gegebenenfalls in 4-Stellung mit einer C₁₋₆-Alkylgruppe oder Mono- oder Polyhydroxyalkylgruppe substituiert sind, ausgewählt sind.

14. Verbindungen nach einem der vorhergehenden Ansprüche, dadurch gekennzeichnet, daß die Halogenatome unter Fluor, Chlor und Brom ausgewählt sind.

15. Verbindungen nach einem der vorhergehenden Ansprüche, dadurch gekennzeichnet, daß die verzweigte Alkylgruppe mit 1 bis 20 Kohlenstoffatomen unter den Gruppen 2-Methylbutyl, 2-Methylpentyl, 1-Methylhexyl und 3-Methylheptyl ausgewählt ist.

16. Verbindungen nach einem der vorhergehenden Ansprüche, dadurch gekennzeichnet, daß die Aminoalkylgruppe unter den Gruppen Aminomethyl, 3-Aminopropyl und 6-Aminohexyl ausgewählt ist.

17. Verbindungen nach einem der vorhergehenden Ansprüche, dadurch gekennzeichnet, daß die Alkenylgruppe 2 bis 6 Kohlenstoffatome aufweist.

18. Verbindungen nach einem der vorhergehenden Ansprüche, dadurch gekennzeichnet, daß die cycloaliphatischen Gruppen mit 3 bis 6 Kohlenstoffatomen unter den Gruppen Cyclopropyl und Cyclohexyl ausgewählt sind.

19. Verbindungen nach Anspruch 1, dadurch gekennzeichnet, daß sie alleine oder im Gemisch ausgewählt sind unter:
4-[3-(1-Adamantyl)-4-methoxyphenylthiomethyl]-benzoesäure.
4-[3-(1-Adamantyl)-4-methoxyphenylsulfinylmethyl]-benzoesäure.
4-[3-(1-Adamantyl)-4-methoxyphenylmethylsulfonyl]-benzoesäure.
4-[3-(1-Adamantyl)-4-methoxyphenylmethylsulfinyl]-benzoesäure.
4-[3-(1-Adamantyl)-4-methoxyphenylsulfonylmethyl]-benzoesäure.
4-[3-(1-Adamantyl)-4-methoxyphenylthiocarboxamido]-benzoesäure.
4-[3-(1-Adamantyl)-4-methoxyphenylmethylthio]-benzoesäure.
4-[3-(1-Adamantyl)-4-methoxyphenylmethylamino]-benzoesäure.
4-[[3-Oxo-3-[3-(1-adamantyl)-4-methoxyphenyl]-1-propinyl]]-benzoesäure.
4-[3-(1-Adamantyl)-4-methoxyethoxymethoxybenzamido]-benzoesäure.
4-[3-(1-Adamantyl)-4-methoxyethoxymethoxybenzoyloxy]-benzoesäure.
(E)-4-[[3-Oxo-3-[3-(1-adamantyl)-4-methoxyethoxymethoxyphenyl]-1-propenyl]]-benzoesäure.
4-[[3-Hydroxy-3-[3-(1-adamantyl)-4-methoxyethoxymethoxyphenyl]-1-propinyl]]-benzoesäure.
4-[3-(1-Adamantyl)-4-(2,2-dimethyl-1,3-dioxolan-4-methyloxy)-phenylcarboxamido]-benzoesäure.
4-[3-(1-Adamantyl)-4-(2,3-dihydroxypropyloxy)-phenylcarboxamido]-benzoesäure.
4-[3-(1-Adamantyl)-4-methoxycarbonylmethyloxyphenylcarboxamido]-benzoesäure.
2-[3-(1-Adamantyl)-4-methoxyphenoxymethyl]-4-thiophencarbonsäure.
2-[3-(1-Adamantyl)-4-methoxyphenylaminomethyl]-4-thiophencarbonsäure.
2-[3-(1-Adamantyl)-4-methoxyphenylthiomethyl]-4-thiophencarbonsäure.
4-[3-(1-Adamantyl)-4-(2,2-dimethyl-1,3-dioxolan-4-methyloxy)-benzoyloxy]-benzoesäure.
4-[3-Hydroxy-3[5-(1-adamantyl)-2,4-dimethoxyethoxymethoxyphenyl]-1-propinyl]-benzoesäure.
4-[3-(1-Adamantyl)-4-methoxyethoxymethoxybenzoylthio]-benzoesäure.
N-Methyl-4-[3-(1-adamantyl)-4-methoxyethoxymethoxyphenylcarboxamido]-benzoesäure.
4-[3-(1-Adamantyl)-4-methoxyethoxymethoxyphenylthiocarboxamido]-benzoesäure.
(E)-4-[[3-Oxo-3-[3-(1-adamantyl)-4-methoxymethoxyphenyl]-1-propenyl]]-benzoesäure.
(E)-4-[[3-Oxo-3-[3-(1-adamantyl)-4-hydroxyphenyl]-1-propenyl]]-benzoesäure.
(E)-4-[[3-Oxo-3-[3-(1-adamantyl)-4-methoxyphenyl]-1-propenyl]]-benzoesäure.
(E)-4-[[3-Oxo-3-[3-(1-adamantyl)-4-(3-hydroxypropyloxy)-phenyl]-1-propenyl]]-benzoesäure.
4-[3-(1-Adamantyl)-4-methoxyethoxymethoxybenzoyloxy]-benzaldehyd.
4-[3-(1-Adamantyl)-4-methoxyethoxymethoxybenzoyloxy]-benzolmethanol.
(E)-N-Ethyl-4-[[3-oxo-3-[3-(1-adamantyl)-4-methoxyethoxymethoxyphenyl]-1-propenyl]]-benzamid.
(E)-N-(4-Hydroxyphenyl)-4-[[3-oxo-3-[3-(1-adamantyl)-4-methoxyethoxymethoxyphenyl]-1-propenyl]]-benzamid.
(E)-4-[[3-Oxo-3-[3-(1-adamantyl)-4-methoxyethoxymethoxyphenyl]-1-propenyl]]-phenol.

20. Verbindungen nach Anspruch 1, dadurch gekennzeichnet, daß sie mindestens eine der folgenden Eigenschaften aufweisen:
- R₁ ist die Gruppe
-COO-R₇ oder -CO-R₆.
- R₃ ist die Gruppe
-Y-(CH₂)p-Y-(CH₂)q-R₈.
-(CH₂)p-(Y)n-(CH₂)q-R₈.
-Y-(CH₂)q-R₈
- X ist eine Bindung der Formel (a), (e), (f), (j) oder (k).
- Ar bedeutet die Gruppe der Formel (a) oder (b).

21. Verbindungen nach einem der vorhergehenden Ansprüche zur Verwendung als Arzneimittel.

22. Verbindungen nach Anspruch 21 zur Verwendung als Arzneimittel, das zur Behandlung von dermatologischen Erkrankungen, rheumatischen Erkrankungen, Erkrankungen der Atemwege, kardiovaskulären Erkrankungen und ophtalmologischen Erkrankungen bestimmt ist.

23. Verwendung mindestens einer Verbindung nach einem der Ansprüche 1 bis 20 zur Herstellung eines Arzneimittels, das zur Behandlung von dermatologischen Erkrankungen, rheumatischen Erkrankungen, Erkrankungen der Atemwege, kardiovaskulären Erkrankungen und ophtalmologischen Erkrankungen bestimmt ist.

24. Pharmazeutische Zusammensetzung, dadurch gekennzeichnet, daß sie in einem pharmazeutisch akzeptablen Träger mindestens eine Verbindung nach einem der Ansprüche 1 bis 20 enthält.

25. Zusammensetzung nach Anspruch 24, dadurch gekennzeichnet, daß die Konzentration der Verbindung(en) nach einem der Ansprüche 1 bis 16 im Bereich von 0,001 bis 5 Gew.-%, bezogen auf die gesamte Zusammensetzung, liegt.

26. Kosmetische Zusammensetzung, dadurch gekennzeichnet, daß sie in einem kosmetisch akzeptablen Träger mindestens eine Verbindung nach einem der Ansprüche 1 bis 20 enthält.

27. Zusammensetzung nach Anspruch 26, dadurch gekennzeichnet, daß die Konzentration der Verbindung(en) nach einem der Ansprüche 1 bis 20 im Bereich von 0,001 bis 3 Gew.-%, bezogen auf die gesamte Zusammensetzung, liegt.

28. Verwendung einer kosmetischen Zusammensetzung nach einem der Ansprüche 26 oder 27 zur Körper- oder Haarhygiene.

## Claims

1. Bicyclic aromatic compounds, characterized in that they correspond to the general formula (I) below: 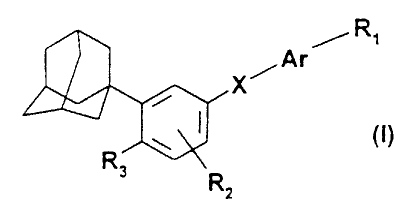 in which:
- R₁ represents
(i) the -CH₃ radical
(ii) the radical -(CH₂)n-O-R₄
(iii) the radical -O-(CH₂)m-(CO)n-R₅
(iv) the radical -CO-R₆
(v) the radical -CO-O-R₇
the values m and n, as well as the various radicals R₄ to R₇, having the meaning given below,
- R₂ represents a hydrogen or halogen atom, a linear or branched alkyl radical having from 1 to 20 carbon atoms, a radical -OR₄ or an -O-CH₂-O-CH₂-CH₂-O-CH₃ radical,
- R₃ represents:
(i) a radical -Y-(CH₂)p-Y-(CH₂)q-R₈
(ii) a radical - (CH₂)p-Y-(CH₂)q-R₈
(iii) a radical -Y-(CH₂)q-R₈
(iv) a radical -CH=CH-(CH₂)r-R₈
(v) a radical -(CH₂)q-R₈
the values p, q and r and the radicals Y and R₈ having the meaning given below,
- X represents the linkages of formulae (a)-(k) below which may be read in both directions: 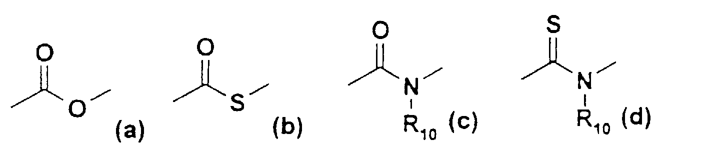 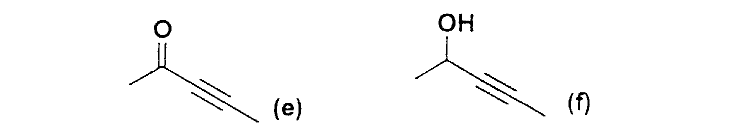 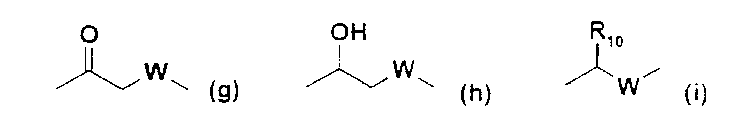 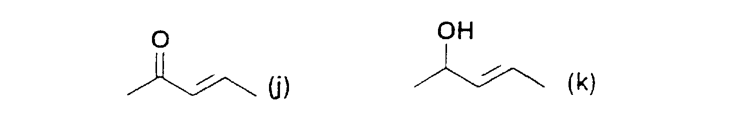
- Ar represents a radical chosen from the radicals of formulae (a)-(f) below: 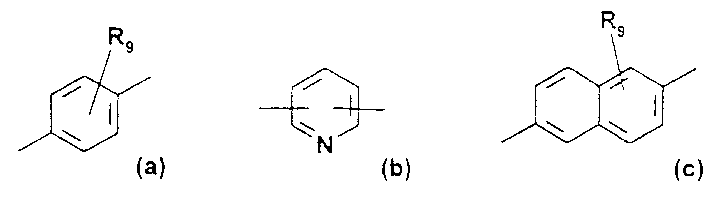 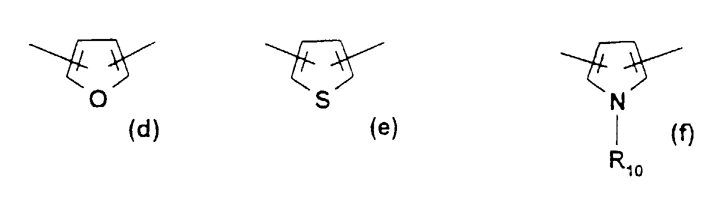
it being understood in all of the above text that:
- m is an integer equal to 1, 2 or 3
- n is an integer equal to 0 or 1
- p is an integer between 1 and 12 inclusive
- q is an integer between 0 and 12 inclusive
- r is an integer between 0 and 10 inclusive
- t is an integer equal to 0, 1 or 2
- Y represents an oxygen atom or a radical S(O)t
- W represents an oxygen atom, a radical S(O)t or a radical N-R₁₀
- R₄ represents a hydrogen atom, a lower alkyl radical or a radical -CO-R₁₁
- R₅ represents a lower alkyl or a heterocycle
- R₆ represents a hydrogen atom, a lower alkyl radical or a radical 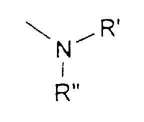 in which R' and R" represent a hydrogen atom, a lower alkyl radical, a mono- or polyhydroxyalkyl radical, an optionally substituted aryl radical or an amino acid or peptide or sugar residue, or alternatively, taken together, form a heterocycle,
- R₇ represents a hydrogen atom, a linear or branched alkyl radical having from 1 to 20 carbon atoms, an alkenyl radical, a mono- or polyhydroxyalkyl radical, an optionally substituted aryl or aralkyl radical or a sugar residue or an amino acid or peptide residue,
- R₈, which may be identical or different, represents a hydrogen atom, a branched alkyl radical having from 1 to 20 carbon atoms, a C3-C6 cycloaliphatic radical, a monohydroxyalkyl radical or a polyhydroxyalkyl radical in which the hydroxyls are optionally protected in methoxy or acetoxy or acetonide form, an aryl radical, an alkynyl radical, a radical -CO-R₆, a radical -CO-O-R₇, an amino alkyl radical whose amine function is optionally substituted with one or two lower alkyl groups, a heterocycle, R₇ having the meaning given above,
- R₉, which may be identical or different, represents a hydrogen or halogen atom, a linear or branched alkyl radical having from 1 to 20 carbon atoms, a radical -OR₄ or an -O-CH₂-O-CH₂-CH₂-O-CH₃ radical,
- R₁₀, which may be identical or different, represents a hydrogen atom or a lower alkyl radical,
- R₁₁ represents a lower alkyl radical given that when:
- X represents the linkage of formula (a), (b), (c), (g), (h), (j) or (k), then R₃ is other than the radical of formula (iii), (iv) or (v) in which R₈ is a hydrogen atom,
- X represents the linkage of formula (a), then R₃ is other than the radical (iii),
- X represents the linkage of formula (b) and R₃ represents the radical of formula (iii) in which Y represents a sulphur atom, then R₈ is other than an aryl radical,
- X represents the linkage of formula (g), (h), (j) or (k) and R₃ represents the radical of formula (v), then R₈ is other than a monohydroxyalkyl or polyhydroxyalkyl radical, or a C₃-C₆ cycloaliphatic radical,
as well as the salts thereof and the optical and geometrical isomers thereof.

2. Compounds according to Claim 1, characterized in that they are in the form of salts of an alkali metal or alkaline-earth metal, or alternatively of zinc or of an organic amine.

3. Compounds according to either of Claims 1 and 2, characterized in that the lower alkyl radicals are chosen from the group consisting of the methyl, ethyl, isopropyl, butyl, tert-butyl, hexyl, nonyl and dodecyl radicals.

4. Compounds according to one of the preceding claims, characterized in that the linear or branched alkyl radicals having from 1 to 20 carbon atoms are chosen from the group consisting of the methyl, ethyl, propyl, 2-ethylhexyl, octyl, dodecyl, hexadecyl and octadecyl radicals.

5. Compounds according to one of the preceding claims, characterized in that the monohydroxyalkyl radicals are chosen from the group consisting of the 2-hydroxyethyl, 2-hydroxypropyl and 3-hydroxypropyl radicals.

6. Compounds according to one of the preceding claims, characterized in that the polyhydroxyalkyl radicals are chosen from the group consisting of the 2,3-dihydroxypropyl, 2,3,4-trihydroxybutyl and 2,3,4,5-tetrahydroxypentyl radicals or the pentaerythritol residue.

7. Compounds according to one of the preceding claims, characterized in that the aryl radical is a phenyl radical optionally substituted with at least one halogen atom, one hydroxyl or one nitro function.

8. Compounds according to one of the preceding claims, characterized in that the aralkyl radicals are chosen from the group consisting of the benzyl or phenethyl radicals, optionally substituted with at least one halogen atom, one hydroxyl or one nitro function.

9. Compounds according to one of the preceding claims, characterized in that the alkenyl radicals are chosen from the group consisting of radicals containing from 1 to 5 carbon atoms and having one or more ethylenic unsaturations, and in particular the allyl radical.

10. Compounds according to one of the preceding claims, characterized in that the sugar residues are chosen from the group consisting of glucose, galactose, mannose and glucuronic acid residues.

11. Compounds according to any one of the preceding claims, characterized in that the amino acid residues are chosen from the group consisting of residues derived from lysine, from glycine or from aspartic acid.

12. Compounds according to any one of the preceding claims, characterized in that the peptide residues are chosen from the group consisting of dipeptide and tripeptide residues.

13. Compounds according to any one of the preceding claims, characterized in that the heterocyclic radicals are chosen from the group consisting of the piperidino, morpholino, pyrrolidino and piperazino radicals, optionally substituted in position 4 with a C₁-C₆alkyl or mono- or polyhydroxyalkyl radical.

14. Compounds according to any one of the preceding claims, characterized in that the halogen atoms are chosen from the group consisting of fluorine, chlorine and bromine.

15. Compounds according to any one of the preceding claims, characterized in that the branched alkyl radical having from 1 to 20 carbon atoms is chosen from the 2-methylbutyl, 2-methylpentyl, 1-methylhexyl and 3-methylheptyl radicals.

16. Compounds according to any one of the preceding claims, characterized in that the aminoalkyl radical is chosen from the aminomethyl, 3-aminopropyl and 6-aminohexyl radicals.

17. Compounds according to any one of the preceding claims, characterized in that the alkynyl radical has from 2 to 6 carbon atoms.

18. Compounds according to any one of the preceding claims, characterized in that the cycloaliphatic radicals of from 3 to 6 carbon atoms are chosen from the cyclopropyl radical and the cyclohexyl radical.

19. Compounds according to Claim 1, characterized in that they are taken, alone or as mixtures, from the group consisting of:
4-[3-(1-Adamantyl)-4-methoxyphenylthiomethyl]-benzoic acid.
4-[3-(1-Adamantyl)-4-methoxyphenylsulphinylmethyl]benzoic acid.
4-[3-(1-Adamantyl)-4-methoxyphenylmethylsulphonyl]benzoic acid.
4-[3-(1-Adamantyl)-4-methoxyphenylmethylsulphinyl]benzoic acid.
4-[3-(1-Adamantyl)-4-methoxyphenylsulphonylmethyl]benzoic acid.
4-[3-(1-Adamantyl)-4-methoxyphenylthiocarboxamido]benzoic acid.
4-[3-(1-Adamantyl)-4-methoxyphenylmethylthio]benzoic acid.
4-[3-(1-Adamantyl)-4-methoxyphenylmethylamino]benzoic acid.
4-[[3-Oxo-3-[3-(1-adamantyl)-4-methoxyphenyl)-1-propynyl]]benzoic acid.
4-[3-(1-Adamantyl)-4-methoxyethoxymethoxybenzamido]benzoic acid.
4-[3-(1-Adamantyl)-4-methoxyethoxymethoxybenzoyloxy]benzoic acid.
(E)-4-[[3-Oxo-3-[3-(1-adamantyl)-4-methoxyethoxymethoxyphenyl)-1-propenyl]]benzoic acid.
4-[[3-Hydroxy-3-[3-(1-adamantyl)-4-methoxyethoxymethoxyphenyl]-1-propynyl]]benzoic acid.
4-[3-(1-Adamantyl)-4-(2,2-dimethyl-1,3-dioxolane-4-methyloxy)phenylcarboxamido]benzoic acid.
4-[3-(1-Adamantyl)-4-(2,3-dihydroxypropyloxy)-phenylcarboxamido]benzoic acid.
4-[3-(1-Adamantyl)-4-methoxycarbonylmethyloxyphenylcarboxamido]benzoic acid.
2-[3-(1-Adamantyl)-4-methoxyphenoxymethyl]-4-thiophenecarboxylic acid.
2-[3-(1-Adamantyl)-4-methoxyphenylaminomethyl]-4-thiophenecarboxylic acid.
2-[3-(1-Adamantyl)-4-methoxyphenylthiomethyl]-4-thiophenecarboxylic acid.
4-[3-(1-Adamantyl)-4-(2,2-dimethyl-1,3-dioxolane-4-methyloxy)benzoyloxy]benzoic acid.
4-[3-Hydroxy-3-[5-(1-adamantyl)-2,4-dimethoxyethoxymethoxyphenyl]-1-propynyl]benzoic acid.
4-[3-(1-Adamantyl)-4-methoxyethoxymethoxybenzoylthio]benzoic acid.
N-Methyl-4-[3-(1-adamantyl)-4-methoxyethoxymethoxyphenylcarboxamido]benzoic acid.
4-[3-(1-Adamantyl)-4-methoxyethoxymethoxyphenylthiocarboxamido]benzoic acid.
(E)-4-[[3-Oxo-3-[3-(1-adamantyl)-4-methoxymethoxyphenyl)-1-propenyl]]benzoic acid.
(E)-4-[[3-Oxo-3-[3-(1-adamantyl)-4-hydroxyphenyl]-1-propenyl]]benzoic acid.
(E)-4-[[3-Oxo-3-[3-(1-adamantyl)-4-methoxyphenyl]-1-propenyl]]benzoic acid.
(E)-4-[[3-Oxo-3-[3-(1-adamantyl)-4-(3-hydroxypropyloxy)phenyl]-1-propenyl]]benzoic acid.
4-[3-(1-Adamantyl)-4-methoxyethoxymethoxybenzoyloxy]benzaldehyde.
4-[3-(1-Adamantyl)-4-methoxyethoxymethoxybenzoyloxy]benzenemethanol.
(E)-N-Ethyl-4-[[3-oxo-3-[3-(1-adamantyl)-4-methoxyethoxymethoxyphenyl]-1-propenyl]]benzamide.
(E)-N-(4-Hydroxyphenyl)-4-[[3-oxo-3-[3-(1-adamantyl)-4-methoxyethoxymethoxyphenyl]-1-propenyl]]benzamide.
(E)-4-[[3-Oxo-3-[3-(1-adamantyl)-4-methoxyethoxymethoxyphenyl)-1-propenyl]]phenol.

20. Compounds according to Claim 1, characterized in that they have at least one of the following characteristics:
- R₁ is the radical
-COO-R₇ or -CO-R₆
- R₃ is the radical
-Y-(CH₂)p-Y-(CH₂)q-R₈
-(CH₂)p-(Y)n-(CH₂)q-R₈
-Y-(CH₂)q-R₈
- X represents a linkage of formula (a), (e), (f), (j) or (k)
- Ar represents the radical of formula (a) or (b).

21. Compounds according to any one of the preceding claims, for a use as a medicinal product.

22. Compounds according to Claim 21, for a use as a medicinal product intended for the treatment of dermatological, rheumatic, respiratory, cardiovascular and ophthalmological complaints.

23. Use of at least one of the compounds defined in Claims 1 to 20 for the manufacture of a medicinal product intended for the treatment of dermatological, rheumatic, respiratory, cardiovascular and ophthalmological complaints.

24. Pharmaceutical composition, characterized in that it comprises, in a pharmaceutically acceptable support, at least one of the compounds as defined in any one of Claims 1 to 20.

25. Composition according to Claim 24, characterized in that the concentration of compound(s) according to one of Claims 1 to 16 is between 0.001 % and 5 % by weight relative to the whole composition.

26. Cosmetic composition, characterized in that it comprises, in a cosmetically acceptable support, at least one of the compounds as defined in any one of Claims 1 to 20.

27. Composition according to Claim 26, characterized in that the concentration of compound(s) according to one of Claims 1 to 20 is between 0.001 % and 3 % by weight relative to the whole composition.

28. Use of a cosmetic composition as defined in either of Claims 26 and 27, for body or hair hygiene.
